(19) Europäisches Patentamt
European Patent Office
Office européen des brevets

(11) EP 2 219 589 B1

(12) FASCICULE DE BREVET EUROPEEN

(45) Date de publication et mention de la délivrance du brevet:
**11.03.2015 Bulletin 2015/11**

(21) Numéro de dépôt: **08854748.4**

(22) Date de dépôt: **14.11.2008**

(51) Int Cl.:
***A61K 8/34*** (2006.01) ***A61K 8/37*** (2006.01)
***A61K 8/41*** (2006.01) ***A61K 8/49*** (2006.01)
***A61Q 5/10*** (2006.01)

(86) Numéro de dépôt international:
**PCT/FR2008/052056**

(87) Numéro de publication internationale:
**WO 2009/068830 (04.06.2009 Gazette 2009/23)**

# (54) COMPOSITION POUR UNE COLORATION D'OXYDATION A PH SUPERIEUR OU EGAL A 8 DES FIBRES KERATINIQUES HUMAINES, COMPRENANT UN ALCOOL GRAS, UN ESTER GRAS ET UN TENSIOACTIF CATIONIQUE, PROCÉDÉ LA METTANT EN OEUVRE ET DISPOSITIF

ZUSAMMENSETZUNG ZUR OXIDATIONSFÄRBUNG VON MENSCHLICHEN KERATINFASERN BEI EINEM PH VON >8, MIT EINEM FETTALKOHOL; EINEM FETTESTER UND EINEM KATIONISCHEN TENSID, VERFAHREN DAMIT UND VORRICHTUNG

COMPOSITION FOR OXIDATION DYEING OF HUMAN KERATIN FIBRES, AT A PH GREATER THAN OR EQUAL TO 8, COMPRISING A FATTY ALCOHOL, A FATTY ESTER AND A CATIONIC SURFACTANT, METHOD USING SAME AND DEVICE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **15.11.2007 FR 0759069**

(43) Date de publication de la demande:
**25.08.2010 Bulletin 2010/34**

(73) Titulaire: **L'Oréal**
**75008 Paris (FR)**

(72) Inventeurs:
- **DEBAIN, Jean-Daniel**
**F-95870 Bezons (FR)**
- **LAGUITTON, Bruno**
**94110 Arcueil (FR)**

(74) Mandataire: **Wattremez, Catherine et al**
**L'Oréal**
**D.I.P.I.**
**25-29 Quai Aulagnier**
**92665 Asnières (FR)**

(56) Documents cités:
**EP-A- 1 426 039** **WO-A-2005/063179**
**DE-A1- 10 020 887** **DE-A1- 19 914 926**
**DE-A1-102004 062 702**

Remarques:
Le dossier contient des informations techniques présentées postérieurement au dépôt de la demande et ne figurant pas dans le présent fascicule.

EP 2 219 589 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition tinctoriale pour la coloration d'oxydation des fibres kératiniques humaines, à un pH supérieur ou égal à 8, comprenant un ou plusieurs précurseurs de colorant d'oxydation, un ou plusieurs alcools gras et esters gras de même qu'un ou plusieurs tensioactifs cationiques, un procédé de coloration des fibres kératiniques dans lequel on applique une telle composition sur lesdites fibres ainsi qu'un dispositif à plusieurs compartiments appropriés pour sa mise en oeuvre.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs précurseurs de colorant d'oxydation, plus particulièrement une ou plusieurs bases d'oxydation éventuellement associées à un ou plusieurs coupleurs.

Habituellement, des bases d'oxydation sont choisies parmi les ortho- ou para-phénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en tes associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Ce mode de coloration peut aussi être combiné à la coloration directe ou semi-permanente. Le procédé classiquement utilisé en coloration directe consiste à appliquer sur les fibres kératiniques des colorants directs qui sont des molécules colorées et colorantes, ayant une affinité pour les fibres, à laisser pauser pour permettre aux molécules de pénétrer, par diffusion, à l'intérieur de la fibre, puis à les rincer.

Les colorants directs généralement employés sont choisis parmi tes colorants directs nitrés benzéniques, anthraquinoniques, nitropyridiniques, azoïques, méthiniques, azométhiniques, xanthéniques, acridiniques, aziniques ou triarylméthaniques.

L'un des inconvénients des procédés de coloration d'oxydation réside dans le fait que la coloration de la composition appliquée sur les fibres varie très fortement entre le début de l'application et celui où la composition est finalement retirée. Ce phénomène, tout à fait classique, est caractéristique du degré d'avancement de la réaction de condensation oxydative précitée, conduisant à l'apparition de l'espèce colorée.

Cependant, en fonction de la nuance choisie, la couleur de la composition est plus ou moins foncée ou plus ou moins chromatique et ne correspond pas toujours parfaitement à la coloration finale de la chevelure, ce qui peut gêner l'utilisateur.

En outre, plus la couleur de la composition est marquée, plus les risques de tâchage de la peau et du cuir chevelu sont augmentés.

Mais l'apparition plus ou moins rapide de la coloration de la composition constitue aussi un inconvénient pour le procédé même de coloration. En effet, le polymère synthétisé dans la composition pendant le temps de pause ne l'est pas dans la fibre kératinique et cela représente une baisse de l'efficacité de la coloration. Il est par conséquent nécessaire d'employer des quantités plus importantes de précurseurs de coloration afin de compenser cette perte en réactifs, occasionnant entre autres une augmentation du coût de la composition.

Un autre inconvénient observé en mettant en oeuvre des procédés de coloration d'oxydation à des pH supérieurs ou égaux à 8, est la conséquence de la présence d'ammoniaque. Cet agent alcalinisant est employé non seulement pour ajuster le pH de la composition tinctoriale mais aussi pour faire gonfler la fibre kératinique afin de favoriser la pénétration des colorants ainsi que de l'agent oxydant dans la fibre où la polycondensation a lieu.

Cet agent étant très volatil, on observe une perte en ammoniac, ce qui n'est pas favorable pour le procédé et occasionne des désagréments à l'utilisateur du fait de l'odeur caractéristique forte, plutôt incommodante de telles compositions.

Il n'est donc pas envisageable de baisser purement et simplement la quantité d'ammoniaque mise en oeuvre dans ces compositions, malgré les risques d'intolérance que l'on peut parfois observer à ces concentrations (irritations du cuir chevelu sous forme de picotements).

L'un des objectifs de la présente invention est donc de proposer des compositions tinctoriales qui limitent les inconvénients précités liés à l'apparition durant l'application, de la coloration de la composition et au dégagement d'ammoniac de la composition lorsque de l'ammoniaque est présente dans la composition, sans toutefois dégrader les propriétés tinctoriales et ni les propriétés d'usage de telles compositions.

Il a en effet été trouvé de façon totalement inattendue que de tels résultats pouvaient être obtenus grâce à l'association, dans des compositions tinctoriales mises en oeuvre à un pH supérieur ou égal à 8, d'un ou plusieurs alcools gras, d'un ou plusieurs esters gras avec un ou plusieurs tensioactifs cationiques.

Dans de telles conditions, on a constaté que l'évolution de la coloration de la composition appliquée était considérablement ralentie au cours de l'application, au point que dans certains cas, la composition ne changeait substantiellement pas de couleur pendant la durée du temps de pause, même lorsque la coloration finale des fibres kératiniques est foncée.

De plus, ce ralentissement de la vitesse de la réaction de condensation oxydative dans la composition n'est avantageusement et de façon inattendue, pas le reflet de la vitesse de coloration au sein de la fibre kératinique même, car il n'est pas nécessaire d'augmenter le temps de pause pour obtenir la coloration souhaitée des fibres.

Il est à noter que cette évolution de la coloration de la composition est particulièrement visible lorsque la composition ne comprend que des colorants d'oxydation. Mais ce phénomène existe toujours lorsque la composition comprend un ou plusieurs colorants directs, même s'il peut être plus ou moins masqué par la couleur apportée par ce ou ces colorants directs.

La composition selon l'invention permet donc d'augmenter l'efficacité du procédé de coloration, en d'autres termes une quantité plus importante de réactif est effectivement utilisée pour colorer la fibre et non la composition, au contraire des compositions classiquement mises en oeuvre.

De plus, cet avantage n'est pas atteint au détriment des autres critères mesurant l'efficacité d'une composition de coloration, que sont notamment la puissance, l'homogénéité, la chromaticité de la coloration résultante, qui restent très bonnes dans le cadre de l'invention.

Ainsi, la composition selon l'invention permet, pour obtenir un niveau de coloration analogue à celui atteint en mettant en oeuvre une composition classique, de baisser la quantité de colorant jusqu'à 20 % en poids par rapport à la quantité employée classiquement.

On a également observé que, de manière inattendue, lorsque de l'ammoniaque est présente dans la composition, la quantité d'ammoniac dégagée de la composition était moins importante par rapport à celle dégagée par des compositions usuelles comprenant une même teneur en ammoniaque.

Les compositions selon l'invention permettent donc de limiter les inconvénients dus à la présence d'ammoniaque, sans qu'il soit nécessaire d'en diminuer la concentration.

Cependant, puisqu'il est possible de limiter le dégagement d'ammoniac, il serait tout à fait envisageable de diminuer la teneur en ammoniaque des compositions sans constater la baisse d'efficacité de la coloration attendue normalement dans de telles circonstances.

Ces buts et d'autres sont atteints par la présente invention qui a donc pour objet une composition (A) dont le pH est supérieur ou égal à 8, pour la coloration des fibres kératiniques humaines, en particulier les cheveux, comprenant, dans un milieu cosmétiquement acceptable :

* un ou plusieurs précurseurs de colorants d'oxydation ;
* un ou plusieurs tensioactifs cationiques ;
* un ou plusieurs esters d'acide gras ;
* un ou plusieurs alcools gras ;
* le rapport pondéral alcool(s) gras / ester(s) d'acide(s) gras étant supérieur à 2 et inférieur à 10;

le rapport pondéral alcool(s) gras / tensioactif(s) cationique(s) étant compris entre 2 et 30.

Elle a aussi pour objet une composition prête à l'emploi (8) dont le pH est supérieur ou égal à 8 comprenant un ou plusieurs précurseurs de colorants d'oxydation, un ou plusieurs tensioactifs cationiques, un ou plusieurs esters d'acides gras, un ou plusieurs alcools gras, un ou plusieurs agents oxydants. Plus particulièrement, cette composition (B) est obtenue par mélange d'une composition tinctoriale (A) précitée, avec une composition oxydante telle ce mélange ait un pH supérieur ou égal à 8.

Elle a de même pour objet un procédé de coloration de fibres kératiniques humaines, en particulier les cheveux, dans lequel on applique la composition prête à l'emploi sur les fibres kératiniques.

Selon une autre variante du procédé selon l'invention, on applique la composition (A) précitée et une composition oxydante successivement, sans rinçage intermédiaire.

Elle concerne enfin un dispositif à plusieurs compartiments comprenant dans au moins un premier compartiment, la composition (A) selon l'invention, et dans au moins un deuxième compartiment, une composition comprenant un ou plusieurs agents oxydants.

D'autres caractéristiques et avantages de l'invention apparaîtront plus clairement à la lecture de la description et des exemples qui suivent.

Il est à noter que dans ce qui va suivre, et à moins d'une autre indication, les bornes d'un domaine de valeurs sont comprises dans ce domaine.

Comme cela a été indiqué auparavant, la composition selon l'invention comprend un ou plusieurs précurseurs de colorants d'oxydation, plus particulièrement une ou plusieurs bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorephénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino-2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer ta pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,5-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl

pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de dérivés pyrazoliques on peut également citer les diamino N,N-dihydropyazolones et notamment celles décrites dans la demande FR 2886136 telles que les composés suivants et leurs sels d'addition: 2,3-Diamino-6,7-dihydro-1H,5H-pyrazolo[1,2-a]pyrazol-1-one, 2-Amino-3-éthylamino-6,7-dihydro-1H,5H-pyrazolo[1,2-a]pyrazol-1-one, 2-Amino-3-isopropylamino-6,7-dihydro-1H,5H-pyrazolo[1,2-a]pyrazol-1-one, 2-Amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-Amino-1,2-diethyl-5-(pyrrolidin-1-yl)-1,2-dihydro-pyrazol-3-one, 4-Amino-5-(3-dimethylamino-pyrrolidin-1-yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques on utilisera préférentiellement te 4,5-diamino 1-(β-hydroxyéthyl) pyrazole et/ou la 2,3-diamino-6,7-dihydro-1H,5H-pyrazolo[1,2,A]pyrazol-1-one et leurs sels d'addition.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représentent chacun avantageusement de 0,0001 à 10% en poids par rapport au poids total de la composition, et de préférence de 0,005 à 5 % en poids par rapport au poids total de la composition.

La composition selon l'invention peut également comprendre un ou plusieurs colorants directs. Ces derniers sont notamment choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de-composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer tes colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri-chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en $C_1$-$C_2$ ; un radical hydroxyalcoxy en $C_2$-$C_4$ ; un radical amino ; un radical amino substitué par un ou deux radicaux allyle, identiques ou différents, en $C_1$-$C_4$ éventuellement porteurs d'au moins un groupement hydroxyle.

Parmi les colorants directs benzéniques utilisables selon l'invention, on peut citer de manière non limitative les composés suivants :

- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène

- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV), et de préférence les composés de formules (I) et (III) :

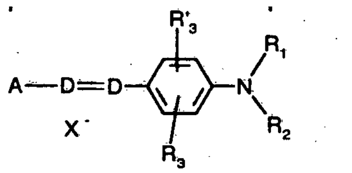

(I)

dans laquelle :

D représente un atome d'azote ou le groupement -CH,

$R_1$ et $R_2$, identiques ou différents, représentent un atome d'hydrogène ; un radical alkyle en $C_1$-$C_4$ pouvant être substitué par un radical -CN, -OH ou -NH2 ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,

$R_3$ et $R'_3$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,

$X^-$ représente un anion de préférence choisi parmi le chlorure, le méthyl sulfate et l'acétate,

A représente un groupement choisi par les structures A1 à A18 suivantes :

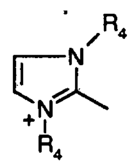

$A_1$

;

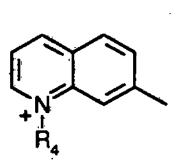

$A_2$

;

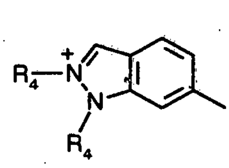

$A_3$

;

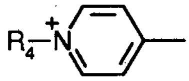 ; 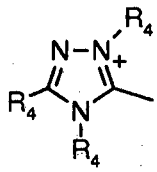 ; 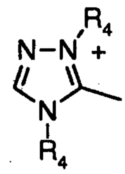 ;

A4 A5 A6

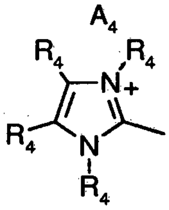 ; 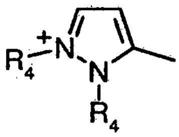 ; 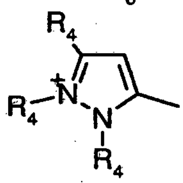 ;

A7 A8 A9

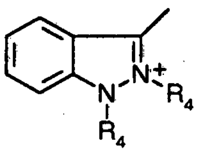 ; 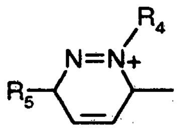 ; 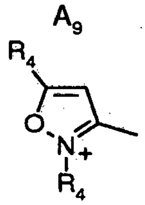 ;

A10 A11 A12

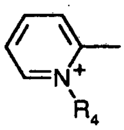 ; 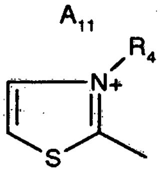 ; 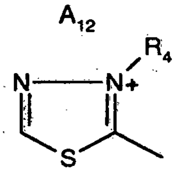 ;

A13 A14 A15

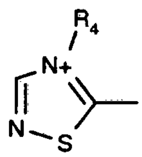 ; 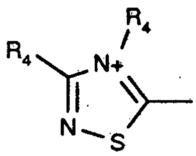 ; 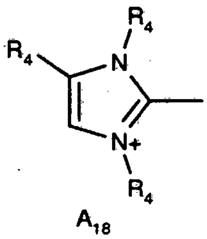

A16 A17 A18

dans lesquelles $R_4$ représente un radical alkyle en $C_1$-$C_4$ pouvant être substitué par un radical hydroxyle et $R_5$ représente un radical alcoxy en $C_1$-$C_4$ ;

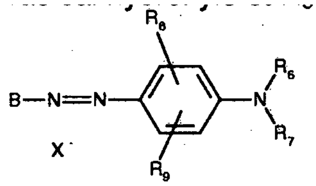 (II)

dans laquelle :

$R_6$ représente un atome d'hydrogène ou un radical alkyle en $C_1$-$C_4$,
$R_7$ représente un atome d'hydrogène, un radical alkyle pouvant être substitué par un radical -CN ou par un groupement amino, un radical 4'-aminophényle ou forme avec $R_6$ un hétérocycle éventuellement oxygéné et/ou azoté pouvant être substitué par un radical alkyle en $C_1$-$C_4$,
$R_8$ et $R_9$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion de préférence choisi parmi le chlorure, le méthyl sulfate et l'acétate,
B représente un groupement choisi par les structures B1 à B6 suivantes :

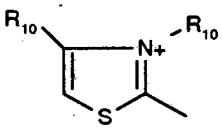 ; 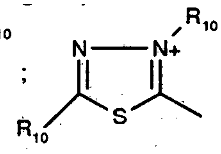 ; 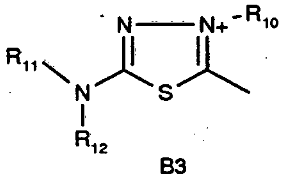 ;

B1 B2 B3

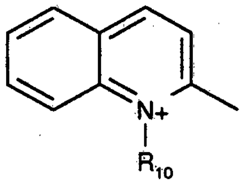 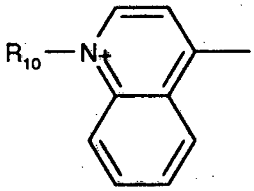 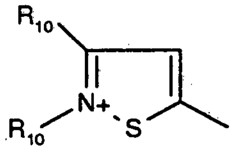

B4 B5 B6

dans lesquelles $R_{10}$ représente un radical alkyle en $C_1$-$C_4$, $R_{11}$ et $R_{12}$, identiques ou différents, représentent un atome d'hydrogène ou un radical alkyle en $C_1$-$C_4$ ;

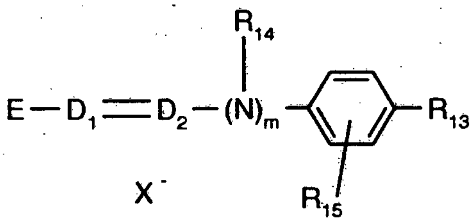

(III)

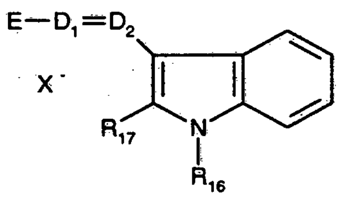

(III')

dans lesquelles :

$R_{13}$ représente un atome d'hydrogène, un radical alcoxy en $C_1$-$C_4$, un atome d'halogène tel que le brome, le chlore, l'iode ou le fluor,
$R_{14}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{15}$ représente un atome d'hydrogène ou d'halogène tel que le brome, le chlore, l'iode ou le fluor,
$R_{16}$ et $R_{17}$, identiques ou différents, représentent un atome d'hydrogène ou un radical alkyle en $C_1$-$C_4$,
$D_1$ et $D_2$, identiques ou différents, représentent un atome d'azote ou le groupement -CH, m = 0 ou 1,
$X^-$ représente un anion de préférence choisi parmi le chlorure, le méthyl sulfate et l'acétate,
E représente un groupement choisi par les structures E1 à E8 suivantes :

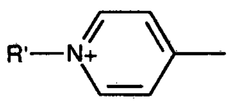 ; 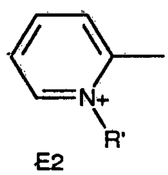 ;

E1 E2

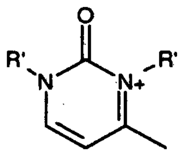

E3

;

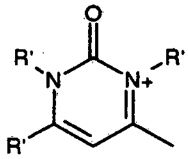

E4

;

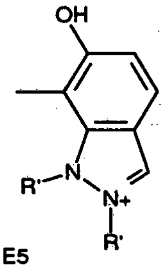

E5

;

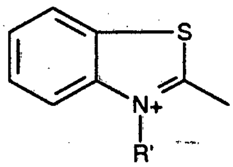

E6

;

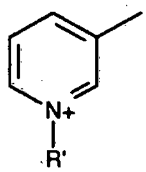

E7

et

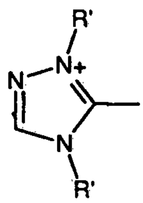

E8

;

dans lesquelles R' représente un radical alkyle en $C_1$-$C_4$ ;
lorsque m = 0 et que $D_1$ représente un atome d'azote, alors E peut également désigner un groupement de structure E9 suivante :

E9

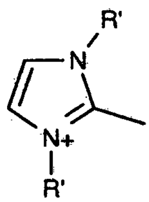

;

dans laquelle R' représente un radical alkyle en $C_1$-$C_4$.

G-N=N-J (IV)

dans laquelle :

le symbole G représente un groupement choisi parmi les structures $G_1$ à $G_3$ suivantes :

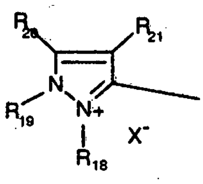

$G_1$

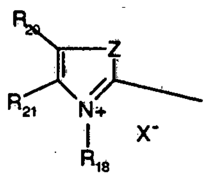

$G_2$

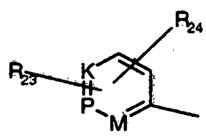

$G_3$

structures $G_1$ à $G_3$ dans lesquelles,

$R_{18}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{19}$ désigne un radical alkyle en $C_1$-$C_4$ ou un radical phényle;
$R_{20}$ et $R_{21}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$, alcoxy en $C_1$-$C_4$, ou $NO_2$, ou forment ensemble dans $G_2$ un cycle benzénique éventuellement substitué par un ou plusieurs radicaux alkyle en $C_1$-$C_4$, alcoxy en $C_1$-$C_4$, ou $NO_2$;
$R_{20}$ peut désigner en outre un atome d'hydrogène;
Z désigne un atome d'oxygène, de soufre ou un groupement $-NR_{19}$;

M représente un groupement -CH, -CR (R désignant alkyle en $C_1$-$C_4$), ou $-NR_{22}(X^-)_r$;

K représente un groupement -CH, -CR (R désignant alkyle en $C_1$-$C_4$), ou $-NR_{22}(X^-)_r$;

P représente un groupement -CH, -CR (R désignant alkyle en $C_1$-$C_4$), ou $-NR_{22}(X^-)_r$; r désigne zéro ou 1 ;

$R_{22}$ représente un atome $O^-$, un radical alcoxy en $C_1$-$C_4$, ou un radical alkyle en $C_1$-$C_4$;

$R_{23}$ et $R_{24}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical $-NO_2$;

$X^-$ représente un anion de préférence choisi parmi le chlorure, l'iodure, le méthyl sulfate, l'éthyl sulfate, l'acétate et le perchlorate;

sous réserve que,

si $R_{22}$ désigne $O^-$, alors r désigne zéro;

si K ou P ou M désignent -N-alkyle $C_1$-$C_4$ $X^-$, alors $R_{23}$ ou $R_{24}$ est ou non différent d'un atome d'hydrogène;

si K désigne $-NR_{22}(X^-)_r$, alors M= P= -CH, -CR;

si M désigne $-NR_{22}(X^-)_r$, alors K= P= -CH, -CR;

si P désigne $-NR_{22}(X^-)_r$ , alors K= M et désignent -CH ou -CR;

si Z désigne un atome de soufre avec $R_{21}$ désignant alkyle en $C_1$-$C_4$, alors $R_{20}$ est différent d'un atome d'hydrogène;

si Z désigne $-NR_{22}$ avec $R_{19}$ désignant alkyle en $C_1$-$C_4$, alors au moins l'un des radicaux $R_{18}$, $R_{20}$ ou $R_{21}$ du groupement de structure $G_2$ est différent d'un radical alkyle en $C_1$-$C_4$;

le symbole J représente :

- (a) un groupement de structure $J_1$ suivante :

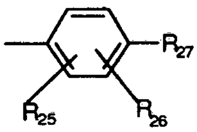

$J_1$

structure $J_1$ dans laquelle,

$R_{25}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{28}$, $-NR_{29}R_{30}$, -NHCOalkyle en $C_1$-$C_4$, ou forme avec $R_{26}$ un cycle à 5 ou 6 chaînons contenant ou non un ou plusieurs hétéroatomes choisis parmi l'azote, l'oxygène ou le soufre;

$R_{26}$ représente un atome d'hydrogène, un atome d'halogène choisi parmi le chlore, le brome, l'iode et le fluor, un radical alkyle en $C_1$-$C_4$, alcoxy en $C_1$-$C_4$,

ou forme avec $R_{27}$ ou $R_{28}$ un cycle à 5 ou 8 chaînons contenant ou non un ou plusieurs hétéroatomes choisis parmi l'azote, l'oxygène ou le soufre;

$R_{27}$ représente un atome d'hydrogène, un radical -OH, un radical $-NHR_{28}$, un radical $-NR_{29}R_{30}$;

$R_{28}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;

$R_{29}$ et $R_{30}$, identiques ou différents, représentent un radical alkyle en $C_1$-$C_4$, un radical monohydroxyalkyle en $C_1$-$C_4$, polyhydroxyalkyle en $C_2$-$C_4$;

- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle,

et notamment un groupement de structure $J_2$ suivante :

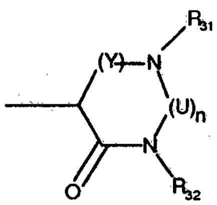

$J_2$

structure $J_2$ dans laquelle,

$R_3$, et $R_{32}$, identiques ou différents, représentent un atome d'hydrogène, un radical alkyle, en $C_1$-$C_4$, un radical phényle;

Y désigne le radical -CO- ou le radical

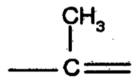 ;

n = 0 ou 1, avec, lorsque n désigne 1, U désigne le radical -CO- .

Dans les structures (I) à (IV) définies ci-dessus le groupement alkyle ou alcoxy en $C_1$-$C_4$ désigne de préférence méthyle, éthyle, butyle, méthoxy, éthoxy.

Parmi les composés de formules (I) et (III), on préfère les composés suivants :

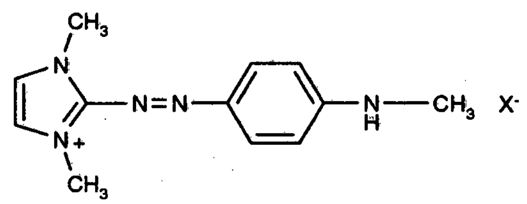

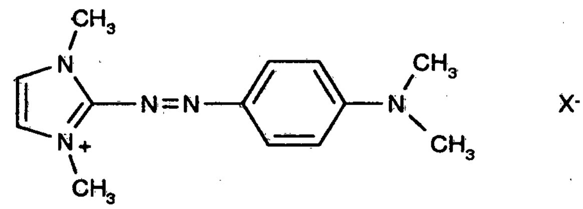

,

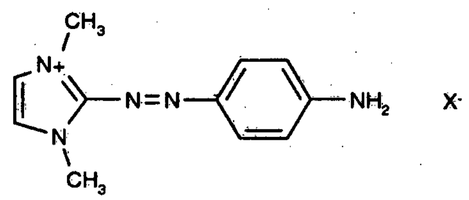

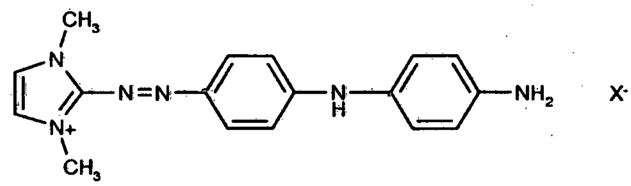

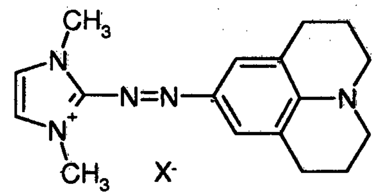

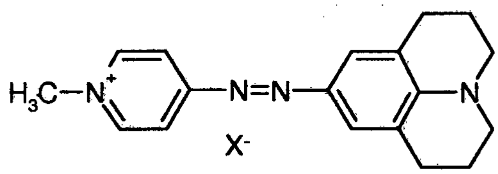

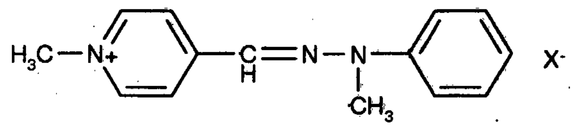

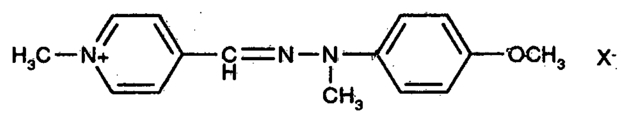

On peut également citer parmi les colorants directs utilisables, les colorants méthiniques tels que te Basic Red 14, et également les colorants directs azoïques notamment choisis parmi les colorants suivants, décrits dans le COLOUR INDEX INTERNATIONAL 3e édition:

- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :

- Disperse Red 15

- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99

ainsi que les composés suivants :

- 1-N-méthylmorpholiniumpropyfamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :

- Basic Blue 17
- Basic Red 2.

Parmi les colorants triarylméthaniques utilisables selon l'invention, on peut citer les composés suivants :

- Basic Green 1
- Basic Violet 3
- Basic Violet 14
- Basic Blue 7
- Basic Blue 26

Parmi les colorants indoaminiques utilisables selon l'invention, on peut citer les composés suivants :

- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment:

| | |
|---|---|
| 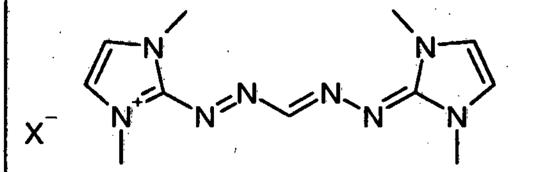 | 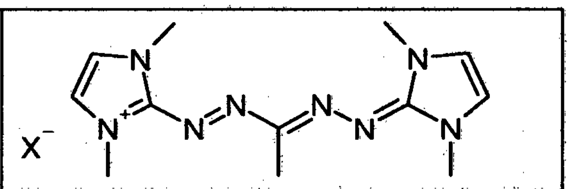 |
| 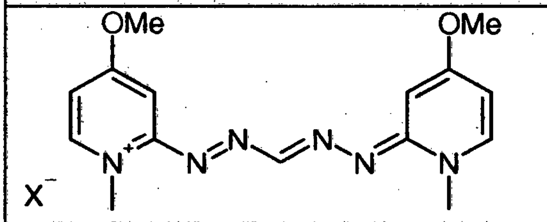 | 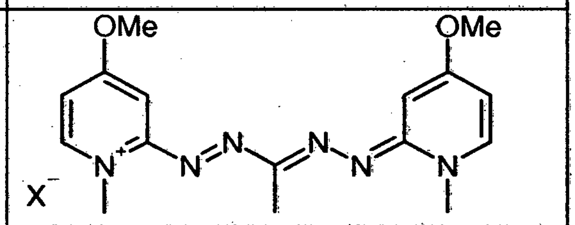 |
| 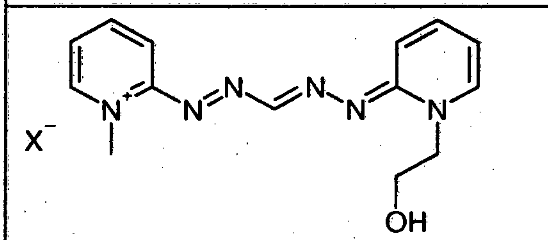 | 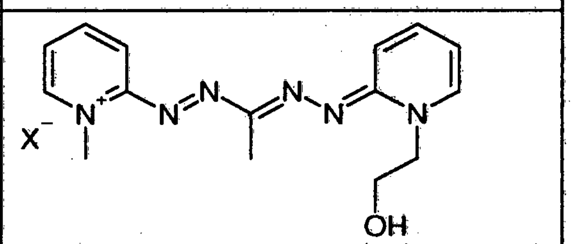 |
| 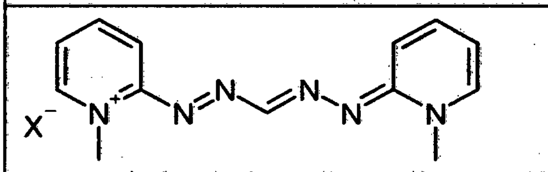 | 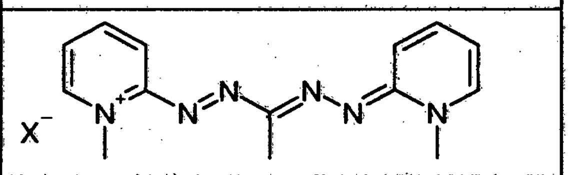 |
| 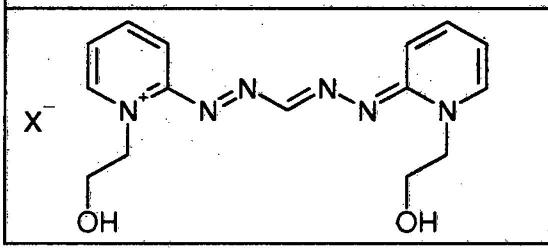 | 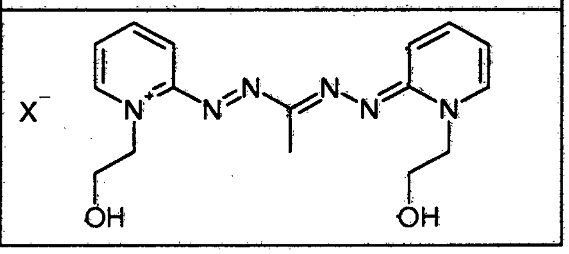 |

X- représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en $C_1$-$C_6$, un noyau phényle éventuellement substitué, ou d'au moins un groupement $N(R')_2$ avec R' identiques ou non, représentant un atome d'hydrogène, un radical alkyle éventuellement substitué en $C_1$-$C_6$, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène un groupement hydroxyle ; un radical alcoxy en $C_1$-$C_2$ ; un radical hydroxyalcoxy en $C_2$-$C_4$ un

radical amino ; un radical amino substitué par un ou deux radicaux alkyle, identiques ou différents, en $C_1$-$C_4$ éventuellement porteurs d'au moins un groupement hydroxyde.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, E167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

Comme indiqué auparavant, la composition selon l'invention comprend un ou plusieurs esters gras.

Par esters gras, au sens de la présente invention, on entend plus particulièrement

des composés choisis parmi, les esters de monoalcools saturés linéaires en $C_1$-$C_{18}$, avec des acides gras monofonctionnels en $C_{14}$-$C_{18}$, ces derniers pouvant être linéaires ou ramifiés, saturés ou insaturés.

La composition selon l'invention présente une teneur en ester(s) gras comprise avantageusement entre 0,3 et 12,5% en poids, par rapport au poids de la composition, de préférence entre 0,5 et 10% en poids et conformément à une variante encore plus préférée de l'invention, de 0,6 à 9% en poids.

La composition selon l'invention comprend de plus un ou plusieurs alcools gras.

Plus particulièrement, ledit alcool gras est choisi parmi les alcools non (poly)oxyalkylénés (l'alkyle ayant 1 à 3 atomes de carbone) et non (poly)glycérolés, comprenant une ou plusieurs chaînes grasses présentant de 10 à 30 atomes de carbone, plus particulièrement de 14 à 22 atomes de carbone et de façon encore plus avantageuse, de 16 à 18 atomes de carbone, saturés ou insaturés, les chaînes grasses étant éventuellement substituées par un ou deux groupements hydroxyle supplémentaires. Lorsque l'alcool est insaturé, il comprend de 1 à 3 doubles liaisons carbone-carbone (-C=C-), conjuguées ou non. De préférence, l'alcool gras est un monoalcool.

A titre d'exemples d'alcools gras, on peut citer l'alcool laurique, cétylique, stéarylique, béhénique, myristique, linoléïque, undécylénique, palmitoléique, linolénique, arachidonique, érucique, isocétylique, isostéarylique, isobéhénylique, l'alcool oléique et leurs mélanges.

De préférence, la composition comprend un ou plusieurs monoalcools gras non (poly)oxyalkylénés et non (poly)glycérolés, comprenant de 14 à 22 atomes de carbone-et plus précisément, de 16 à 18 atomes de carbone, saturés.

Selon un mode de réalisation de l'invention, la composition présente une teneur en alcool(s) gras comprise entre 3 et 25 % en poids, par rapport au poids de la composition, de préférence entre 5 et 20 % en poids et conformément à une variante encore plus préférée de l'invention, de 6 à 18 % en poids.

Il est à noter que selon une caractéristique très avantageuse de l'invention, le ou les esters gras et le ou les alcools gras sont présents en quantités telles que le rapport pondéral alcool(s) gras / ester(s) gras est supérieur à 2 et inférieur à 10. De préférence, le rapport pondéral alcool(s) gras / ester(s) gras est compris entre 3 et 9.

La composition selon l'invention comprend par ailleurs un ou plusieurs tensioactifs cationiques.

Plus particulièrement, ces tensioactifs sont choisis parmi les composés comprenant une ou plusieurs charges

cationiques et une ou plusieurs chaînes alkyle ou alcényle, en $C_{12}$-$C_{30}$ portant éventuellement une ou plusieurs fonctions ester ou amide. Il est par ailleurs précisé que les tensioactifs cationiques entrant dans la composition selon l'invention ne portent pas de charge anionique. Ces tensioactifs ne sont donc pas des espèces amphotères ou zwitterioniques.

A titre d'exemples de tensioactifs cationiques convenables, on peut mentionner :

(i) les sels d'ammonium quaternaires de formule (V) suivante :

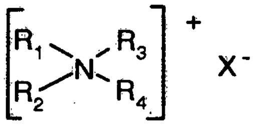

(V)

dans laquelle

$X^-$ est un anion choisi par exemple dans le groupe des halogénures (chlorure, bromure ou iodure) ou alkyl($C_2$-$C_6$)sulfates plus particulièrement méthylsulfate, des phosphates, des alkyl-ou-alkylarylsulfonates, des anions dérivés d'acide organique tel que l'acétate ou le lactate, et

(1) les radicaux $R_1$ à $R_3$, identiques ou différents, représentent un radical aliphatique, plus particulièrement alkyle, linéaire ou ramifié, en $C_1$-$C_4$, ou un radical aryle ou alkylaryle, le radical alkyle étant éventuellement porteur d'un groupement alcoxy, alkylamide,
$R_4$ désigne un radical alkyle, linéaire ou ramifié, en $C_{16}$-$C_{30,}$ de préférence en $C_{18}$-$C_{22}$ ; ou
(2) les radicaux $R_1$ et $R_2$, identiques ou différents, représentent un radical aliphatique, plus particulièrement alkyle, linéaire ou ramifié, en $C_1$-$C_4$, ou un radical que aryle ou alkylaryle, le radical alkyle étant éventuellement porteur d'un groupement alcoxy, alkylamide ou hydroxyalkyle, en $C_1$-$C_4$ ;
$R_3$ et $R_4$, identiques ou différents, désignent un radical alkyle ou alcényle, linéaire ou ramifié, en $C_{12}$-$C_{30}$, ledit radical comprenant éventuellement au moins une fonction ester ou amide ;

Avantageusement, le tensioactif cationique selon l'option (1) précitée est un sel (par exemple chlorure) de béhényl triméthyl ammonium, et selon l'option (2) un sel (par exemple chlorure) de stéaramidopropyl diméthyl (myristylacétate) ammonium commercialisé sous la dénomination «CERAPHYL 70» par la société VAN DYK.
(ii) les sels d'ammonium quaternaire de l'imidazolinium de formule (VI) suivante :

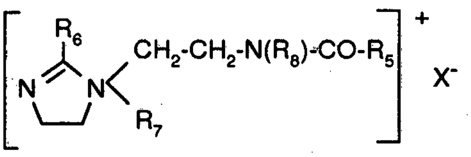

(VI)

dans laquelle

$R_5$ représente un radical alcényle ou alkyle en $C_{12}$-$C_{30}$,
$R_6$ représente un atome d'hydrogène, un radical alkyle en $C_1$-$C_4$ ou un radical alcényle ou alkyle en $C_{12}$-$C_{30}$,
$R_7$ représente un radical alkyle en $C_1$-$C_4$, $R_8$ représente un atome d'hydrogène, un radical alkyle en $C_1$-$C_4$,
X est un anion X notamment choisi dans le groupe des halogénures, phosphates, acétates, lactates, alkylsulfates, alkyl-ou-alkyfaryfsulfonates.

De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO;
(iii) les sels de diammonium quaternaire de formule (VII) :

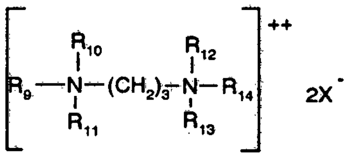

(VII)

dans laquelle

$R_9$ désigne un radical alkyle ou alcényle en $C_{12}$-$C_{30}$,
$R_{10}$, $R_{11}$, $R_{12}$, $R_{13}$ et $R_{14}$, identiques ou différents sont choisis parmi l'hydrogène ou un radical alkyle en $C_1$-$C_4$, et X est un anion notamment choisi dans le groupe des halogénures, acétates, phosphates, nitrates et méthylsulfates.

De tels sels de diammonium quaternaire comprennent notamment le dichlorure de propanesuif diammonium.
(iv) les sels d'ammonium quaternaire contenant au moins une fonction ester de formule (VIII) suivante :

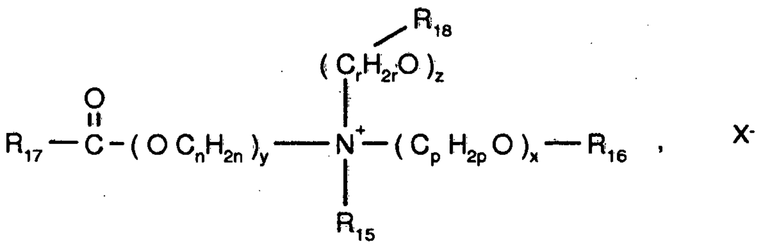

(VIII)

dans laquelle :

- $R_{15}$ est choisi parmi les radicaux alkyle en $C_1$-$C_6$, hydroxyalkyle en $C_1$-$C_6$, dihydroxyalkyle en $C_2$-$C_6$ ;
- $R_{16}$ est choisi parmi le radical $H_{19}$-CO-, un radical $R_{20}$ alkyle ou alcényle en $C_1$-$C_{22}$ linéaire ou ramifié, un atome d'hydrogène,
- $R_{18}$ est choisi parmi le radical $R_{21}$-CO-, un radical $R_{22}$ alkyle ou alcényle en $C_1$-$C_6$ linéaires ou ramifiés, un atome d'hydrogène,
- $R_{17}$, $R_{19}$ et $R_{21}$, identiques ou différents, sont choisis parmi les radicaux alkyle ou alcényle en $C_{12}$-$C_{22}$, linéaires ou ramifiés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- $X^-$ est un anion simple ou complexe, organique ou inorganique.

On utilise plus particulièrement les sels d'ammonium de formule (VIII) dans laquelle :

- $R_{15}$ désigne un radical méthyle ou éthyle,
- x et y sont égaux à 1 ;
- z est égal à 0 ou 1 ;
- n, p et r sont égaux à 2 ;
  $R_{16}$ est choisi parmi le radical $R_{19}$-CO-, les radicaux méthyle, éthyle, alkyle ou alcényle en $C_{14}$-$C_{22}$ ; l'atome d'hydrogène ;
- $R_{17}$, $R_{19}$ et $R_{21}$, identiques ou différents, sont choisis parmi les radicaux alkyle ou alcényle en $C_7$-$C_{21}$, linéaires ou ramifiés, saturés ou insaturés ;
- $R_{18}$ est choisi parmi le radical $R_{21}$-CO-, l'atome d'hydrogène.

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

(v) ou leurs mélanges.

Conformément à un mode de réalisation particulièrement avantageux de l'invention, la composition comprend, à titre de tensioactif cationique, un ou plusieurs tensioactifs cationiques de formule (V), et de préférence un ou plusieurs tensioactifs cationiques de formule (V) correspondant à l'option (1).

Selon un mode de réalisation de l'invention, la composition présente une teneur en tensioactif(s) cationique(s) comprise entre 0,01 et 25 % en poids, par rapport au poids de la composition, de préférence entre 0,05 et 20 % en poids et conformément à une variante particulière de l'invention, de 0,1 à 18 % en poids.

Le milieu cosmétiquement acceptable comprend généralement de l'eau ou un mélange d'eau et d'un ou plusieurs solvants organiques.

A titre de solvant organique, on peut par exemple citer, les alcanols, linéaires ou ramifiés, en $C_2$-$C_4$, tels que l'éthanol et l'isopropanol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Le ou les solvants peuvent être présents dans des proportions allant de préférence de 1 à 40% en poids par rapport au poids total de la composition tinctoriale, et encore plus préférentiellement de 5 à 30% en poids.

Les compositions selon l'invention peuvent également comprendre divers additifs classiquement utilisés dans le domaine de la coloration des fibres kératiniques humaines.

La composition peut ainsi comprendre des agents épaississants minéraux ou organiques, et en particulier des polymères non ioniques, anioniques cationiques ou amphotères, éventuellement associatifs ; des agents matifiants ou opacifiants comme les oxydes de titane ; des agents tensioactifs anioniques, non ioniques, amphotères ou zwittérioniques ; des agents de pénétration, des agents séquestrants, comme l'éthylènediamine tétraacétique ou ses sels ; des agents dispersants ; des agents filmogènes ; des agents conservateurs ; des vitamines ; des parfums des céramides ; des silicones non cationiques, volatiles ou non ; des filtres UV ; des huiles végétales ou minérales.

Les additifs tels que définis précédemment peuvent être présents en quantité comprise pour chacun d'eux entre 0,01 et 40% en poids, de préférence entre 0,1 et 30% en poids par rapport au poids total de la composition.

La composition peut également refermer un ou plusieurs agents antioxydants comme l'acide ascorbique, l'acide érythorbique. Elle peut aussi comprendre un ou plusieurs agents réducteurs comme le sulfite, bisulfite ou métabisulfite d'ammonium ou encore le thiolactate d'ammonium.

Habituellement, les teneurs en agent réducteurs et antioxydants, lorsqu'ils sont présents, varie de 0,005 à 12% en poids par rapport au poids total de la composition, de préférence de 0,1 à 8% en poids.

La composition selon l'invention peut également éventuellement comprendre un ou plusieurs polymères substantifs, cationiques ou amphotères.

Le caractère substantif (c'est à dire l'aptitude au dépôt sur les cheveux) des polymères est classiquement déterminé au moyen du test décrit par Richard J. Crawford, Journal of the Society of Cosmetic Chemists, 1980, 31 - (5) - pages 273 à 278 (révélation par colorant acide Red 80).

Ces polymères substantifs sont notamment décrits dans la demande de brevet EP 557203.

Plus particulièrement, on peut mettre en oeuvre des polymères choisis parmi les dérivés cationiques cellulosiques, les homopolymères d'halogénure de diméthyldiallylammonium (par exemple le Merquat 100) et tes copolymères d'halogénure de diméthyldiallylammonium et d'acide (méth)acrylique ; les homopolymères et copolymères d'halogénure de méthacryloyloxyéthyl-triméthylammonium ; les polymères polyammonium quaternaires ; les polymères de vinylpyrrolidone à motifs cationiques ; leurs mélanges.

Parmi les polymères substantifs du type copolymère d'halogénure de diméthyldiallylammonium utilisables selon l'invention, on peut citer en particulier :

- les copolymères de chlorure de diallyldiméthylammonium et d'acide acrylique comme celui de proportions (80/20 en poids) vendu sous la dénomination Merquat 280 par la société Calgon;
- les copolymères du chlorure de diméthyldiallylammonium et de l'acrylamide vendus sous les dénominations Merquat 550 et Merquat S par la société Merck.

Parmi les polymères substantifs du type polymère d'halogénure de méthacryloyloxyéthyltriméthylammonium utilisables selon l'invention, on peut citer en particulier les produits qui sont dénommés dans le dictionnaire CTFA (5ème édition, 1993) "Polyquaternium 37", "Polyquaternium 32" et "Polyquaternium 35", qui correspondent respectivement, en ce qui concerne le "Polyquaternium 37", au poly(chlorure de méthacryloyloxyéthyltriméthyl-ammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du

copolymère de méthacryloyloxyéthyltriméthylammonium et de méthacryloyloxyéthyldiméthylacétylammonium, vendu sous la dénomination Plex 7525L par la société Rohm GmbH.

Les polymères substantifs du type polyammonium quaternaire utilisables selon l'invention sont les suivants :

- les polymères préparés et décrits dans le brevet français 2 270 846, constitués de motifs récurrents répondant à la formule (IX) suivante :

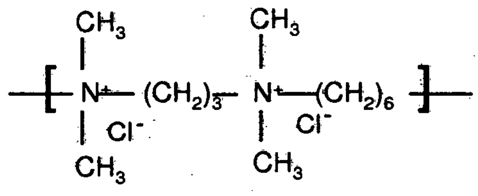

(IX)

notamment ceux dont le poids moléculaire, déterminé par chromatographie par perméation de gel, est compris entre 9500 et 9900 ;

- les polymères préparés et décrits dans le brevet français 2 270 846, constitués de motifs récurrents répondant à la formule (X) suivante :

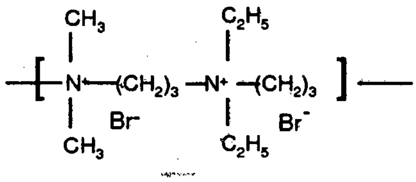

(X)

notamment ceux dont le poids moléculaire, déterminé par chromatographie par perméation de gel, est d'environ 1200 ;

- les polymères décrits et préparés dans les brevets US 4 157 388, 4 390 689, 4 702 906, 4 719 282, et constitués de motifs récurrents répondant à la formule (XI) suivante :

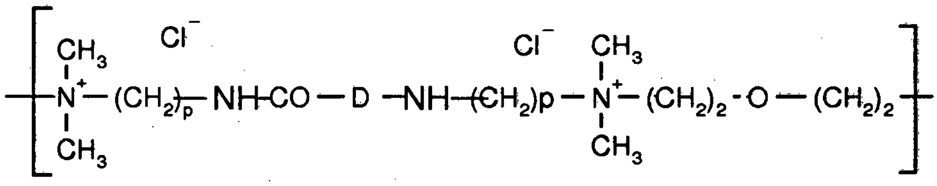

(XI)

dans laquelle p désigne un nombre entier variant de 1 à 6 environ, D peut être nul ou peut représenter un groupement $-(CH_2)_rCO-$ dans lequel r désigne un nombre égal à 4 ou à 7, et notamment ceux dont la masse moléculaire est inférieure à 100 000, de préférence inférieure ou égale à 50 000 ; de tels polymères sont notamment vendus par la société Miranol sous les dénominations "Mirapol A15", "Mirapol AD1", "Mirapol AZ1" et "Mirapol 175".

Parmi les polymères de Vinylpyrrolidone (PVP) à motifs cationiques utilisables conformément à l'invention, on peut citer en particutier :

a) les polymères de Vinylpyrrolidone comportant des motifs Méthacrylate de diméthylaminoéthyle ; on peut citer parmi ceux-ci :

- le copolymère Vinylpyrrolidone / Méthacrylate de diméthylaminoéthyle (20/80 en poids) vendu sous la dénomination commerciale COPOLYMER 845 par la société I.S.P.
- les copolymères Vinylpyrrolidone / Méthacrylate de diméthylaminoéthyle quaternisés par du sulfate de diéthyle, vendus sous les dénominations GAFQUAT 734, 755, 755 S et 755 L par la société I.S.P.
- les PVP / Méthacrylate de diméthylaminoéthyle / Polyuréthane hydrophile, vendus sous la dénomination commerciale PECOGEL GC-310 par la société U.C.I.B. ou encore sous les dénominations AQUAMERE C 1031 et C 1511 par la société BLAGDEN CHEMICALS,

- les PVP / Méthacrylate de diméthylaminoéthyle / Oléfine en C8 à C16, quaternisés ou non quarternisés, vendus sous les dénominations GANEX ACP 1050 à 1057, 1062 à 1069, 1079 à 1086, par la société I.S.P.
- le PVP / Méthacrylate de diméthylaminoéthyle / Vinylcaprolactame, vendu sous la dénomination GAFFIX VC 713 par la société I.S.P.

b) les polymères de Vinylpyrrolidone comportant des motifs Méthacrylamidopropyltriméthylammonium ( M.A.P.T.A.C. ), parmi lesquels on peut citer notamment :

- les copolymères Vinylpyrrolidone / M.A.P.T.A.C., vendus sous les dénominations commerciales GAFQUAT ACP 1011 et GAFQUAT HS 100 par la société I:S.P.

c) les polymères de Vinylpyrrolidone comportant des motifs Méthylvinylimidazolium, et parmi lesquels on peut citer plus particulièrement :

- les PVP / Chlorure de méthylvinylimidazolium, vendus sous les dénominations LUVIQUAT FC 370, FC 550, FC 905, HM 552 par la société B.A.S.F.
- le PVP / Chlorure de méthylvinylimidazolium / Vinylimidazole, vendu sous la dénomination LUVIQUAT 8155 par la société B.A.S.F.
- le PVP / Méthosulfate de méthylvinylimidazolium, vendu sous la dénomination LUVIQUAT MS 370 par la société B.A.S.F.

Parmi les polysiloxanes cationiques on peut notamment citer ceux décrits dans la demande de brevet EP-A-0557203, de la page 8 ligne 48 à la page 11 ligne 9, et plus particulièrement encore les produits comprenant l'Amodiméthicone" (dénomination C.T.F.A.) de formule (XII) suivante :

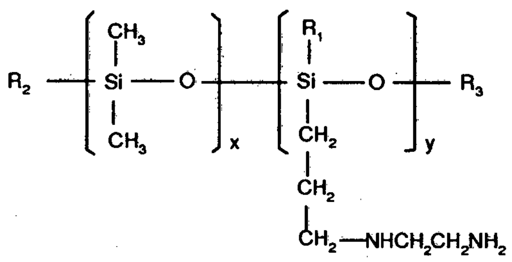

(XII)

formule dans laquelle $R_1$, $R_2$, $R_3$, indépendamment les uns des autres représentent un atome d'hydrogène, un radical hydroxyle, un radicale alkyle en $C_1$-$C_4$, un radical alcoxy en $C_1$-$C_4$ ; x, y dépendant de la masse molaire du composé et de son taux de substitution en groupements aminés.

Lorsqu'ils sont présents, la concentration en polymères substantifs cationiques ou amphotères peut varier entre 0,01 et 10% environ par rapport au poids de la composition, et de préférence entre 0,1 et 5% en poids par rapport au poids de la composition.

Le pH des compositions (A) et (B) conformes à l'invention est supérieur à 8, généralement compris entre 8 et 12 exclu, et de préférence entre 9 et 11 exclu. Il peut être ajusté à la valeur désirée au moyen d'un ou plusieurs agents acidifiants ou d'un ou plusieurs agents alcalinisants habituellement utilisés dans le domaine.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide, citrique, l'acide lactique, les acides sulfoniques.

Parmi les agents alcalinisants on peut citer, à titre d'exemple, l'ammoniaque, tes carbonates alcalins, les alcanolamines telles que les mono-, di- et triéthanolamines ainsi que leurs dérivés, les hydroxydes de sodium ou de potassium et les composés de formule suivante :

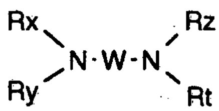

dans laquelle W est un reste propylène éventuellement substitué par un groupement hydroxyle ou un radical alkyle en $C_1$-$C_6$ ; Rx, Ry, Rz et Rt, identiques ou différents, représentent un atome d'hydrogène, un radical alkyle en $C_1$-$C_6$ ou

hydroxyalkyle en $C_1$-$C_6$.

De préférence, l'agent alcalinisant est l'ammoniaque, éventuellement associé à un ou plusieurs autres agents alcalinisants.

La composition (A) selon l'invention peut comprendre également un ou plusieurs agents oxydants. On parle dans ce cas de composition prête à l'emploi (B).

En particulier, la composition prête à l'emploi (B) est obtenue par mélange extemporané avant l'application, d'une composition tinctoriale (A) précédemment décrite, avec au moins une composition comprenant un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Selon une première variante, le procédé selon l'invention consiste à appliquer sur les fibres kératiniques humaines, ces dernières pouvant être sèches ou humides, la composition prête à l'emploi (B) qui vient d'être détaillée et qui est obtenue par mélange extemporané avant l'application, d'une composition tinctoriale dépourvue d'agent oxydant (A) avec une composition oxydante.

Cette composition oxydante, dont le milieu, cosmétiquement acceptable comprend au moins de l'eau, comprend également un ou plusieurs agents oxydants tels que définis plus haut.

Concernant les solvants organiques éventuellement présents dans la composition oxydante, on pourra se reporter à la liste indiquée auparavant dans le cadre du descriptif de la composition tinctoriale.

Habituellement, le pH de la composition oxydante est inférieur à 7.

La composition oxydante peut se présenter sous la forme d'une solution, une émulsion ou un gel.

Elle peut éventuellement comprendre un ou plusieurs additifs utilisés classiquement dans le domaine de la coloration des fibres kératiniques humaines, en fonction de la forme galénique souhaitée. On pourra là encore se reporter à la liste des additifs donnée plus haut.

Généralement, le taux de dilution de la composition tinctoriale est tel que la composition résultante puisse être facilement appliquée sur les fibres kératiniques à colorer, tout en restant localisée à l'endroit où elle a été appliquée, afin d'éviter les problèmes causés par les coulures de composition hors de la zone à traiter.

Plus particulièrement, le taux de dilution (composition tinctoriale/composition oxydante) varie de 0,8 à 3, et de préférence de 1 à 2.

Selon une deuxième variante de l'invention, le procédé selon l'invention consiste à appliquer, la composition (A) dépourvue d'agent oxydant, et une composition oxydante successivement, sans rinçage intermédiaire.

On peut ainsi appliquer la composition oxydante puis la composition tinctoriale ou l'inverse.

Conformément à un mode de réalisation plus particulier de cette variante, on applique sur les fibres kératiniques humaines, sèches ou humides, la composition oxydante avant la composition (A) tinctoriale dépourvue d'agent oxydant.

Selon cette possibilité, les conditions sont telles qu'à la suite de l'application des deux compositions, le pH du mélange présent sur les fibres est supérieur ou égal à 8, plus particulièrement compris entre 8 et 12 et de préférence entre 8 et 11.

Quelle que soit la variante retenue, le mélange présent sur les fibres est laissé en place pour une durée, en général, de l'ordre de 1 minute à 1 heure, de préférence de 10 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

Les exemples suivants servent à illustrer l'invention sans toutefois présenter un caractère limitatif.

<u>EXEMPLE 1</u>

On prépare la composition (1a) suivante :

Tableau 1a

| Ingrédients | Concentration (en g% Matière Active) |
|---|---|
| Alcool cétylique | 6,25 |
| Alcool stéarylique | 6,25 |

(suite)

| Ingrédients | Concentration (en g% Matière Active) |
|---|---|
| Palmitate de stéaryle | 0,75 |
| Stéarate de stéaryle | 1,17 |
| Behentrimonium chloride | 2,97 |
| paraphénylènediamine | 0,63 |
| résorcinol | 0,196 |
| 2,4-diaminophénoxyéthanol | 0,98 |
| Acide érythorbique | 0,25 |
| Métabisulfite de sodium | 0,45 |
| Ammoniaque | 4,11 |
| Pentasodium pentetate | 0,08 |
| Eau | qs 100 |

La composition ainsi obtenue donne lieu à une formule crème onctueuse de couleur blanche.

Elle est ensuite mélangée à une composition oxydante à 20 volumes de -peroxyde d'hydrogène avec un ratio pondéral composition / composition oxydante de 1/1,5 puis appliquée sur une mèche de cheveux 90% blancs naturels pendant 30 minutes à température ambiante (environ 20°C).

A l'issue du temps de pause, la mèche est rincée à l'eau puis séchée.

Le pouvoir tinctorial de la composition (1 a) est comparé à celui obtenu en mettant en oeuvre dans les mêmes conditions la composition (1b) suivante :

Tableau 1b :

| Ingrédient | Concentration (en g% Matière Active) |
|---|---|
| alcool, oléique polyglycérolé à 2 moles de glycérol | 4 |
| alcool oléique polyglycérolé à 4 moles de glycérol | 5,69 |
| acide oléique | 3 |
| amine oléique (2OE) | 7 |
| laurylamino succinamate de diéthylaminopropyle | 3 |
| alcool oléique | 5 |
| diéthanolamine d'acide oléique | 12 |
| propylène glycol | 3,5 |
| éthanol | 7 |
| dipropylène glycol | 0,5 |
| monométhyl éther de propylène glycol | 9 |
| acétate d'ammonium | 0,8 |
| paraphénylènediamine | 0,63 |
| résorcinol | 0,196 |
| 2,4-diaminophénoxyéthanol | 0,98 |
| Acide érythorbique | 0,25 |
| Métabisulfite de sodium | 0,45 |
| EDTA | 0,2 |
| Ammoniaque | 4,11 |

(suite)

| Ingrédient | Concentration (en g% Matière Active) |
|---|---|
| eau | qs100 |

La coloration est mesurée au moyen d'un colorimètre Minolta CM2600d dans le système CIE Lab (illuminant D65, angle 10°, composante spéculaires incluses).

Les résultats sont rassemblés dans le tableau suivant :

| Composition | Couleur | L* |
|---|---|---|
| composition (1a) | Noir bleu | 16,27 |
| composition (1b) | Noir bleu | 18,59 |

On constate que la coloration obtenue en mettant en oeuvre la compositions (1a) selon l'invention est plus intense que celle obtenue en mettant en oeuvre la composition (1 b).

De plus, la coloration de la composition selon l'invention (1a) après mélange avec l'oxydant, se révèle très sélectivement sur la fibre et très peu, voire pas du tout, dans la composition restant sur les cheveux et également dans les eaux de rinçage de la mèche de cheveux.

Au contraire, la coloration de la composition (1b) obtenue après mélange avec l'oxydant augmente très fortement, coloration retrouvée dans le jus de coloration.

Enfin, le dégagement d'ammoniac de la composition selon l'invention (composition 1 a) est toujours inférieur à celui de la composition de référence (composition 1b) comme cela ressort du tableau n°1c ci-dessous.

En outre, on a fait évaluer par 3 personnes le niveau de dégagement d'ammoniac pour 10 couples de compositions (1a) et (1b), immédiatement après le mélange avec l'agent oxydant (t = 0) et après 15 minutes (t = 15 mns) d'une part dans le bol de mélange, et d'autre part après avoir appliqué chaque composition sur tête.

Après évaluation, il apparaît que tous les évaluateurs ont constaté un dégagement d'ammoniac nettement inférieur dans le cadre des compositions prêtes à l'emploi de l'invention aussi bien dans le bol contenant les mélanges à t=0 et à t=15 minutes que sur têtes à t=0 et à t=15 minutes.

<u>EXEMPLE 2</u>

On procède comme pour l'exemple 1 et l'on compare les propriétés tinctoriales des compositions suivantes (2a) et (2b) suivantes :

Tableau 2a

| Ingrédients | Concentration (en g% Matière Active) |
|---|---|
| Alcool cétylique | 8,12 |
| Alcool stéarylique | 8,12 |
| Palmitate de stéaryle | 0,97 |
| Stéarate de stéaryle | 1,53 |
| Behentrimonium chloride | 3,89 |
| toluène-2,5-diamine | 0,61 |
| 2,4-diaminophenoxyéthanol | 0,01 |
| 6-hydroxyindole | 0,04 |
| p-méthylaminophénal sulfate | 0,15 |
| 2-amino-3-hydroxypiridine | 0,02 |
| résorcinol | 0,7 |
| m-aminophénol | 0,165 |
| p-aminophénol | 0,45 |

(suite)

| Ingrédients | Concentration (en g% Matière Active) |
|---|---|
| acide érythorbique | 0,25 |
| métabisulfite de sodium | 0,45 |
| Ammoniaque | 4,94 |
| Pentasodium pentetate | 0,08 |
| Eau | qs 100 |

Tableau 2b :

| Ingrédients | Concentration (en g% Matière Active) |
|---|---|
| alcool oléique polyglycérolé à 2 moles de glycérol | 4 |
| alcool oléique polyglycérolé à 4 moles de glycérol | 5,69 |
| acide oléique | 3 |
| amine oléique (2OE) | 7 |
| laurylamino succinamate de diéthylaminopropyle | 3 |
| alcool oléique | 5 |
| diéthanolamide d'acide oléique | 12 |
| propylène glycol | 3,5 |
| éthanol | 7 |
| dipropylène glycol | 0,5 |
| monométhyl éther de propylène glycol | 9 |
| acétate d'ammonium | 0,8 |
| toluene-2,5-diamine | 0,61 |
| 2,4-diaminophénoxyéthanol | 0,01 |
| 6-hydroxyindole | 0,04 |
| p-méthylaminophénol sulfate | 0,15 |
| 2-amino-3-hydroxypiridine | 0,02 |
| résorcinol | 0,7 |
| m-aminophénol | 0,165 |
| p-aminophénol | 0,45 |
| Acide érythorbique | 0,25 |
| Métabisulfite de sodium | 0,45 |
| EDTA | 0,2 |
| Ammoniaque | 4,11 |
| eau | qs100 |

Les résultats sont rassemblés dans le tableau ci-dessous :

Tableau 2c

| Composition | Couleur | L* |
|---|---|---|
| composition (2a) | Blond foncé doré | 23,14 |
| composition (2b) | Blond foncé doré | 26,14 |

On constate que la coloration obtenue en mettant en oeuvre la composition (2a) selon l'invention est plus intense que celle obtenue en mettant en oeuvre la composition (2b).

De plus, la coloration de la composition selon l'invention (2a) après mélange avec l'oxydant, se révèle très sélectivement sur la fibre et très peu, voire pas du tout, dans la composition restant sur les cheveux et également dans les eaux de rinçage de la mèche de cheveux.

Au contraire, la coloration de la composition (2b) obtenue après mélange avec l'oxydant augmente très fortement, coloration retrouvée aussi dans les eaux de rinçage.

Enfin, on constate que le dégagement d'ammoniac de la composition selon l'invention issue de la composition (2a) est moins important que celui de la composition issue de la composition de référence (2b).

EXEMPLE 3

On procède comme pour l'exemple 1 et l'on compare les propriétés tinctoriales des compositions suivantes (3a) et (3b) suivantes :

Tableau 3a

| Ingrédients | Concentration (en g% Matière active) |
|---|---|
| Alcool cétylique | 9,37 |
| Alcool stéarylique | 9,37 |
| Palmitate de stéaryle | 1,12 |
| Stéarate de stéaryle | 1,76 |
| Behentrimonium chloride | 4,46 |
| toluène-2,5-diamine | 0,026 |
| 2,4-diaminophénoxyéthanol | 0,008 |
| résorcinol | 0,1 |
| m-aminophénol | 0,02 |
| p-aminophenol | 0,039 |
| Acide erythorbique | 0,25 |
| Metabisulfite de sodium | 0,45 |
| Ammoniaque | 5,76 |
| Pentasodium pentetate | 0,08 |
| Eau | Qs 100 |

Tableau n°3b :

| Ingrédients | Concentration (en g% Matière Active) |
|---|---|
| alcool oléique polyglycérolé à 2 moles de glycérol | 4 |
| alcool oléique polyglycérolé à 4 moles de glycérol | 5,69 |
| acide oléique | 3 |
| amine oléique (2OE) | 7 |
| laurylamino succinamate de diéthylaminopropyle | 3 |

(suite)

| Ingrédients | Concentration (en g% Matière Active) |
|---|---|
| alcool oléique | 5 |
| diéthanolamide d'acide oléique | 12 |
| propylène glycol | 3,5 |
| éthanol | 7 |
| dipropylène glycol | 0,5 |
| monométhyl éther de propylène glycol | 9 |
| acétate d'ammonium | 0,8 |
| toluène-2,5-diamine | 0,026 |
| 2,4-diaminophénoxyéthanol | 0,008 |
| résorcinol | 0,1 |
| m-aminophénol | 0,02 |
| p-aminophénol | 0,039 |
| Acide érythorbique | 0,25 |
| Métabisulfite de sodium | 0,45 |
| EDTA | 0,2 |
| Ammoniaque à 20% d'ammoniac | 5,76 |
| eau | qs100 |

Les résultats sont rassemblés dans le tableau ci-dessous :

Tableau 3c

| Composition | Couleur | L* |
|---|---|---|
| composition (3a) | Blond très clair aux reflets cendré doré | 50,42 |
| composition (3b) | Blond très clair aux reflets cendré doré | 52,75 |

On constate que la coloration obtenue en mettant en oeuvre la composition (3a) selon l'invention est plus intense que celle obtenue en mettant en oeuvre la composition (3b).

En outre, la coloration de la composition selon l'invention (3a) après mélange avec l'oxydant, se révèle très sélectivement sur la fibre et très peu, voire pas du tout, dans la composition restant sur les cheveux et également dans les eaux de rinçage de la mèche de cheveux, alors que ce n'est pas le cas de la composition (3b).

Enfin, on constate que le dégagement d'ammoniac de la composition selon l'invention issue de la composition (3a) est moins important que celui de la composition issue de la composition de référence (3b).

EXEMPLE 4

On prépare les compositions suivantes (les quantités sont exprimées en gramme %) :

| | Composition comparative | Composition de référence |
|---|---|---|
| Glycérine | 5 | 5 |
| EDTA | 0,3 | 0,3 |
| Acide ascorbique | 0,1 | 0,1 |
| Sulfite de sodium | 0,3 | 0,3 |

(suite)

| | Composition comparative | Composition de référence |
|---|---|---|
| Ammoniaque à 20% | 7 | 7 |
| Alcool myristique | 0,9 | 1,8 |
| Alcool cetylique | 6,6 | 13,2 |
| Huile de ricin | 0,7 | 0,7 |
| Stéarate de glycéryle | 1,2 | 1 |
| Alcool laurique 23 OE | 0,3 | 0,3 |
| Alcool Cetylstéarylique 33 OE | 0,9 | 0,9 |
| Stearyltrimethyl ammonium chloride | 3,6 | 3,6 |
| Palmitate d'isopropyle | 2,6 | 2 |
| Oxyde de titane | 0,13 | 0,13 |
| Para-toluene diamine sulfate | 0,63 | 0,63 |
| Résorcine | 0,98 | 0,98 |
| 2,4-diaminophénoxyéthanol | 0,196 | 0,196 |
| Eau | Qsp 100 | Qsp 100 |
| pH | 10,1 | 10,1 |
| Rapport pondéral alcool gras/ester d'acide gras | 1,97 | 5,00 |
| Rapport pondéral alcool gras/tensioactif cationique | 2,08 | 4,17 |

Chaque composition est mélangée à un même volume d'une composition oxydante à 20 volumes de peroxyde d'hydrogène.

On a alors procédé à deux types de mesures pour chacun des mélanges :

1) On a mesuré au moyen d'un colorimètre l'évolution de la couleur de chacun des mélanges pendant un temps de pause de 30 minutes, toutes les minutes, en plaçant une partie de chaque mélange dans une cellule apposée à la cellule de lecture du colorimètre empêchant toute interférence de la mesure avec la lumière extérieure de la pièce où est réalisée la mesure.

2) On a appliqué l'autre partie de chaque mélange sur des mèches de cheveux naturels à 90% blancs, pendant 30 minutes à température ambiante (environ 20°C).

A l'issue du temps de pause, la mèche est rincée à l'eau puis séchée.

Les mesures ont été réalisées au moyen d'un colorimètre Minolta CM2600d dans le système CIE Lab (illuminant D65, angle 10°, composante spéculaires incluses).

<u>Evolution de la couleur des mélanges pendant le temps de pause</u>

On rappelle que DE, qui évalue ici la puissance tinctoriale du mélange, correspond au calcul suivant :

$$DE = [L^{*2} + a^{*2} + b^{*2}]1/2$$

où L*, a* et b* correspondent aux valeurs des coordonnées dans le système CIE L*a*b.

Plus DE est élevée, plus le mélange est coloré.

| | Composition de référence | | | | Composition comparative | | | |
|---|---|---|---|---|---|---|---|---|
| | L* | a* | b* | DE | L* | a* | b* | DE |
| Référence | 66,62 | -0,5 | 15,46 | - | 66,14 | -0,41 | 9,26 | - |
| 0 min | 64,12 | 0,97 | 12,72 | 3,99 | 57,2 | 0,82 | 11,07 | 9,21 |
| 1 | 65,21 | 0,89 | 13,7 | 2,64 | 57,23 | 0,42 | 11,71 | 9,28 |
| 2 | 64,52 | 0,92 | 14,41 | 2,74 | 56,68 | 0,41 | 12,74 | 10,11, |
| 3 | 63,72 | 0,97 | 15 | 3,28 | 56,1 | 0,45 | 13,67 | 11,00 |
| 4 | 63,05 | 1,02 | 15,44 | 3,88 | 55,58 | 0,48 | 14,39 | 11,77 |
| 5 min | 62,38 | 1,08 | 15,81 | 4,54 | 55,11 | 0,53 | 15,05 | 12,49 |
| 6 | 61,8 | 1,14 | 16,18 | 5,14 | 54,63 | 0,57 | 15,58 | 13,16 |
| 7 | 61,23 | 1,18 | 16,49 | 5,74 | 54,17 | 0,6 | 16,09 | 13,81 |
| 8 | 60,69 | 1,26 | 16,74 | 6,32 | 53,73 | 0,65 | 16,54 | 14,43 |
| 9 | 60,17 | 1,3 | 16,97 | 6,86 | 53,3 | 0,71 | 16,92 | 14,99 |
| 10 min | 59,69 | 1,36 | 17,17 | 7,38 | 52,9 | 0,72 | 17,3 | 15,53 |
| 11 | 59,20 | 1,39 | 17,36 | 7,89 | 52,52 | 0,79 | 17,58 | 16,00 |
| 12 | 58,76 | 1,46 | 17,51 | 8,35 | 52,14 | 0,83 | 17,85 | 16,47 |
| 13 | 58,31 | 1,48 | 17,64 | 8,81 | 51,77 | 0,86 | 18,12 | 16,93 |
| 14 | 57,87 | 1,52 | 17,76 | 9,27 | 51,41 | 0,89 | 18,36 | 17,36 |
| 15 min | 57,44 | 1,56 | 17,86 | 9,71 | 51,07 | 0,95 | 18,55 | 17,75 |
| 16 | 57,04 | 1,59 | 17,96 | 10,12 | 50,73 | 0,97 | 18,72 | 18,13 |
| 17 | 56,64 | 1,62 | 18,01 | 10,51 | 50,41 | 1,02 | 18,86 | 18,48 |
| 18 | 56,27 | 1,65 | 18,06 | 10,88 | 50,08 | 1,03 | 19,03 | 18,85 |
| 19 | 55,87 | 1,66 | 18,12 | 11,28 | 49,77 | 1,05 | 19,15 | 19,18 |
| 20 min | 55,51 | 1,69 | 18,15 | 11,64 | 49,47 | 1,09 | 19,25 | 19,49 |
| 21 | 55,15 | 1,71 | 18,17 | 11,99 | 49,16 | 1,1 | 19,35 | 19,80 |
| 22 | 54,79 | 1,73 | 18,18 | 12,34 | 48,85 | 1,14 | 19,44 | 20,12 |
| 23 | 54,44 | 1,74 | 18,19 | 12,68 | 48,56 | 1,17 | 19,49 | 20,40 |
| 24 | 54,07 | 1,76 | 18,19 | 13,04 | 48,27 | 1,2 | 19,55 | 20,68 |
| 25 min | 53,73 | 1,78 | 18,17 | 13,37 | 47,97 | 1,22 | 19,6 | 20,97 |
| 26 | 53,38 | 1,79 | 18,13 | 13,70 | 47,68 | 1,24 | 19,62 | 21,23 |
| 27 | 53,03 | 1,8 | 18,11 | 14,04 | 47,39 | 1,28 | 19,64 | 21,50 |
| 28 | 52,7 | 1,83 | 18,07 | 14,35 | 47,11 | 1,29 | 19,66 | 21,75 |
| 29 | 52,37 | 1,82 | 18,05 | 14,67 | 46,83 | 1,31 | 19,67 | 22,01 |
| 30 min | 52,04 | 1,83 | 17,97 | 14,98 | 46,53 | 1,34 | 19,65 | 22,26 |
| Référence : composition seule (avant mélange avec la composition oxydante). | | | | | | | | |

On constate que la coloration du mélange comparatif est considérablement plus importante que dans le cas du mélange de référence

Coloration des mèches

| | L* | a* | b* | DE* |
|---|---|---|---|---|
| Mèche vierge | 55,01 | 1,78 | 13,34 | - |
| Composition de référence | 17,26 | -0,08 | -0,39 | 40,22 |
| Composition comparative | 18,21 | -0,1 | 0,18 | 39,14 |

On constate que les mèches colorées à partir de la composition de référence sont aussi bien colorées (légèrement meilleure) que celles qui le sont à partir de la composition comparative.

On en conclut de ces deux séries de résultats, que les compositions selon l'invention permettent de colorer les cheveux au moins aussi bien que les compositions connues, tout en diminuant significativement la coloration du mélange de la composition avec la composition oxydante, ainsi que son évolution, pendant le temps de pause.

## Revendications

**1.** Composition tinctoriale (A) pour la coloration des fibres kératiniques humaines, comprenant, à un pH supérieur ou égal à 8, dans un milieu cosmétiquement acceptable,

* un ou plusieurs précurseurs de colorant d'oxydation ;
* un ou plusieurs tensioactifs cationiques ;
* un ou plusieurs esters d'acide gras choisis parmi les esters de monoalcools saturés linéaires en $C_1$-$C_{18}$, avec des acides gras monofonctionnels en $C_{14}$-$C_{18}$, linéaires, ramifiés, saturés ou insaturés ;
* un ou plusieurs alcools gras,
* le rapport pondéral alcool(s) gras / ester(s) d'acide gras étant supérieur à 2 et inférieur à 10 ;
* le rapport pondéral alcool(s) gras / tensioactif(s) cationique(s) étant compris entre 2 et 30.

**2.** Composition selon l'une quelconque des revendications précédentes, **caractérisée en ce que** la composition comprend un ou plusieurs colorants directs.

**3.** Composition selon l'une quelconque des revendications précédentes, **caractérisée en ce que** la teneur en ester(s) gras est comprise entre 0,3 et 12,5 % en poids, par rapport au poids de la composition.

**4.** Composition selon l'une quelconque des revendications précédentes, **caractérisée en ce que** l'alcool gras choisi parmi les monoalcools non (poly)oxyalkylénés et non (poly)glycérolés, comprenant de 14 à 22 atomes de carbone, de préférence comprenant de 16 à 18 atomes de carbone, saturés.

**5.** Composition selon l'une quelconque des revendications précédentes, **caractérisée en ce que** la teneur en alcool(s) gras est comprise entre 3 et 25 % en poids, par rapport au poids de la composition.

**6.** Composition selon l'une quelconque des revendications précédentes, **caractérisée en ce que** le tensioactif cationique est choisi parmi :

(i) les sels d'ammonium quaternaires de formule (V) suivante :

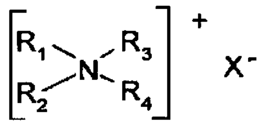

(V)

dans laquelle

$X^-$ est un anion et

(1) les radicaux $R_1$ à $R_3$, identiques ou différents, représentent un radical aliphatique, linéaire ou ramifié, en $C_1$-$C_4$, ou un radical aryle ou alkylaryle, le radical alkyle étant éventuellement porteur d'un groupement alcoxy, alkylamide, $R_4$ désigne un radical alkyle, linéaire ou ramifié, en $C_{16}$-$C_{30}$ ; ou
(2) les radicaux $R_1$ et $R_2$, identiques ou différents, représentent un radical aliphatique, linéaire ou ramifié, en $C_1$-$C_4$, ou un radical que aryle ou alkylaryle,

le radical alkyle étant éventuellement porteur d'un groupement alcoxy, alkylamide ou hydroxyalkyle, en $C_1$-$C_4$;
$R_3$ et $R_4$, identiques ou différents, désignent un radical alkyle ou alcényle, linéaire ou ramifié, en $C_{12}$-$C_{30}$, ledit radical comprenant éventuellement au moins une fonction ester ou amide ;

(ii) les sels d'ammonium quaternaire de l'imidazolinium de formule (VI) suivante :

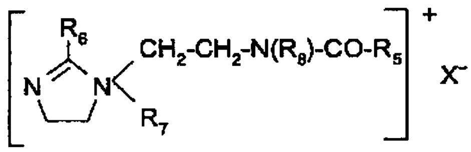 $X^-$ (VI)

dans laquelle

$R_5$ représente un radical alcényle ou alkyle en $C_{12}$-$C_{30}$,
$R_6$ représente un atome d'hydrogène, un radical alkyle en $C_1$-$C_4$ ou un radical alcényle ou alkyle en $C_{12}$-$C_{30}$,
$R_7$ représente un radical alkyle en $C_1$-$C_4$, $R_8$ représente un atome d'hydrogène, un radical alkyle en $C_1$-$C_4$,
X est un anion ;

(iii) les sels de diammonium quaternaire de formule (VII) :

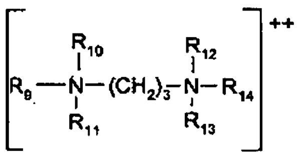 $2X^-$ (VII)

dans laquelle

$R_9$ désigne un radical alkyle ou alcényle en $C_{12}$-$C_{30}$,
$R_{10}$, $R_{11}$, $R_{12}$, $R_{13}$ et $R_{14}$, identiques ou différents sont choisis parmi l'hydrogène ou un radical alkyle en $C_1$-$C_4$, et X est un anion ;

(iv) les sels d'ammonium quaternaire contenant au moins une fonction ester de formule (VIII) suivante :

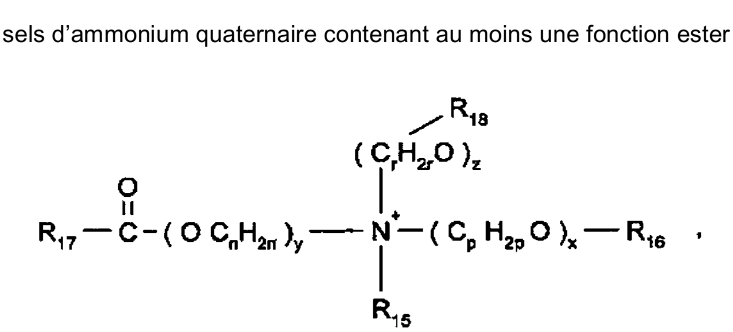 $X^-$ (VIII)

dans laquelle :

• $R_{15}$ est choisi parmi les radicaux alkyle en $C_1$-$C_6$, hydroxyalkyle en $C_1$-$C_6$, dihydroxyalkyle en $C_2$-$C_6$ ;

- $R_{16}$ est choisi parmi le radical $R_{19}$-CO-, un radical $R_{20}$ alkyle ou alcényle en $C_1$-$C_{22}$ linéaire ou ramifié, un atome d'hydrogène,
- $R_{18}$ est choisi parmi le radical $R_{21}$-CO-, un radical $R_{22}$ alkyle ou alcényle en $C_1$-$C_6$ linéaires ou ramifiés, un atome d'hydrogène,
- $R_{17}$, $R_{19}$ et $R_{21}$, identiques ou différents, sont choisis parmi les radicaux alkyle ou alcényle en $C_{12}$-$C_{22}$, linéaires ou ramifiés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- $X^-$ est un anion simple ou complexe, organique ou inorganique ;

sous réserve que la somme x + y + z vaut de 1 à 15, que lorsque x vaut 0 alors $R_{16}$ désigne $R_{20}$ et que lorsque z vaut 0 alors $R_{18}$ désigne $R_{22}$ ;
(v) ou leurs mélanges.

**7.** Composition selon l'une quelconque des revendications précédentes, **caractérisée en ce que** le tensioactif cationique est de formule (V), de préférence de formule (V) option (1).

**8.** Composition selon l'une quelconque des revendications précédentes, **caractérisée en ce que** la teneur en tensioactif(s) cationique(s) est comprise entre 0,01 et 25 % en poids, par rapport au poids de la composition.

**9.** Composition selon l'une quelconque des revendications précédentes, **caractérisée en ce que** le rapport pondéral alcool(s) gras / ester(s) gras étant compris entre 3 et 9.

**10.** Composition selon l'une quelconque des revendications précédentes, **caractérisée en ce qu'**elle comprend un ou plusieurs agents oxydants.

**11.** Composition selon l'une quelconque des revendications précédentes, **caractérisée en ce qu'**elle présente un pH compris entre 8 et 12 exclu, de préférence entre 9 et 11 exclu.

**12.** Procédé de coloration de fibres kératiniques humaines, **caractérisé en ce que** l'on applique sur les fibres kératiniques, sèches ou humides, une composition prête à l'emploi (B) obtenue par mélange extemporané avant l'application, d'une composition tinctoriale (A) selon l'une quelconque des revendications 1 à 9, avec au moins une composition comprenant un ou plusieurs agents oxydants.

**13.** Procédé de coloration de fibres kératiniques humaines, **caractérisé en ce que** l'on applique la composition (A) selon l'une quelconque des revendications 1 à 9 en présence d'une composition oxydante ; la composition oxydante étant appliquée avant ou après ladite composition (A), sans rinçage intermédiaire.

**14.** Dispositif à plusieurs compartiments comprenant dans au moins un premier compartiment, la composition (A) selon l'une quelconque des revendications 1 à 9, et dans au moins un deuxième compartiment, une composition comprenant un ou plusieurs agents oxydants.

## Patentansprüche

**1.** Färbezusammensetzung (A) zum Färben von menschlichen Keratinfasern, die bei einem pH-Wert von mehr als oder gleich 8 in einem kosmetisch unbedenklichen Medium

* eine oder mehrere Oxidationsfarbstoffvorstufen;
* ein oder mehrere kationische Tenside;
* einen oder mehrere Fettsäureester, die aus Estern von linearen, gesättigten $C_1$-$C_{18}$-Monoalkoholen mit linearen, verzweigten, gesättigten oder ungesättigten monofunktionellen $C_{14}$-$C_{18}$-Fettsäuren ausgewählt sind;
* einen oder mehrere Fettalkohole
umfasst,
* wobei das Gewichtsverhältnis von Fettalkohol(en) zu Fettsäureester(n) mehr als 2 und weniger als 10 beträgt;
* wobei das Gewichtsverhältnis von Fettalkohol(en) zu kationischem Tensid bzw. kationischen Tensiden zwischen 2 und 30 liegt.

**2.** Zusammensetzung nach dem vorhergehenden Anspruch, **dadurch gekennzeichnet, dass** die Zusammensetzung einen oder mehrere Direktfarbstoffe umfasst.

**3.** Zusammensetzung nach einem der vorhergehenden Ansprüche, **dadurch gekennzeichnet, dass** der Gehalt an Fettsäureester(n) zwischen 0,3 und 12,5 Gew.-%, bezogen auf das Gewicht der Zusammensetzung, liegt.

**4.** Zusammensetzung nach einem der vorhergehenden Ansprüche, **dadurch gekennzeichnet, dass** der Fettalkohol aus nicht (poly)oxyalkylenierten und nicht (poly)glycerinierten gesättigten Fettmonoalkoholen mit 14 bis 22 Kohlenstoffatomen und vorzugsweise 16 bis 18 Kohlenstoffatomen ausgewählt ist.

**5.** Zusammensetzung nach einem der vorhergehenden Ansprüche, **dadurch gekennzeichnet, dass** der Gehalt an Fettalkohol(en) zwischen 3 und 25 Gew.-%, bezogen auf das Gewicht der Zusammensetzung, liegt.

**6.** Zusammensetzung nach einem der vorhergehenden Ansprüche, **dadurch gekennzeichnet, dass** das kationische Tensid aus:

(i) den quartären Ammoniumsalzen der folgenden Formel (V):

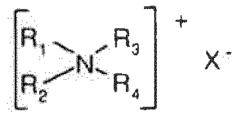

(V)

worin

$X^-$ ein Anion ist und

(1) die Reste $R_1$ bis $R_3$ gleich oder verschieden sind und für einen linearen oder verzweigten aliphatischen $C_1$-$C_4$-Rest oder einen Aryl- oder Alkylalkylrest stehen, wobei der Alkylrest gegebenenfalls eine Alkoxy- oder Alkylamidgruppe tragen kann,
$R_4$ für einen linearen oder verzweigten $C_{16}$-$C_{30}$-Alkylrest steht; oder
(2) die Reste $R_1$ und $R_2$ gleich oder verschieden sind und für einen linearen oder verzweigten aliphatischen $C_1$-$C_4$-Rest oder einen Aryl- oder Alkylalkylrest stehen, wobei der Alkylrest gegebenenfalls eine Alkoxy-, Alkylamid- oder Hydroxyalkylgruppe mit 1 bis 4 Kohlenstoffatomen tragen kann;

$R_3$ und $R_4$ gleich oder verschieden sind und für einen linearen oder verzweigten Alkyl- oder Alkenylrest mit 12 bis 30 Kohlenstoffatomen stehen, wobei der Rest gegebenenfalls mindestens eine Ester- oder Amidfunktion enthalten kann;

(ii) den quartären Imidazolinium-Ammoniumsalzen der folgenden Formel (VI):

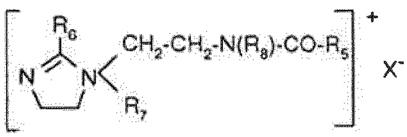

(VI)

worin

$R_5$ für einen Alkenyl- oder Alkylrest mit 12 bis 30 Kohlenstoffatomen steht,
$R_6$ für ein Wasserstoffatom, einen $C_1$-$C_4$-Alkylrest oder einen Alkenyl- oder Alkylrest mit 12 bis 30 Kohlenstoffatomen steht,
$R_7$ für einen $C_1$-$C_4$-Alkylrest steht,
$R_8$ für ein Wasserstoffatom oder einen $C_1$-$C_4$-Alkylrest steht,
X ein Anion ist;

(iii) den quartären Diammoniumsalzen der Formel (VII):

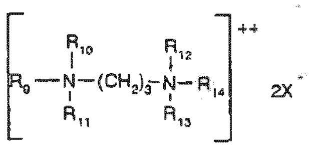 (VII)

worin

$R_9$ für einen Alkyl- oder Alkenylrest mit 12 bis 30 Kohlenstoffatomen steht,
$R_{10}$, $R_{11}$, $R_{12}$, $R_{13}$ und $R_{14}$ gleich oder verschieden sind und unter Wasserstoff oder einem $C_1$-$C_4$-Alkylrest ausgewählt sind und X ein Anion ist;

(iv) den quartären Ammoniumsalzen mit mindestens einer Esterfunktion der folgenden Formel (VIII):

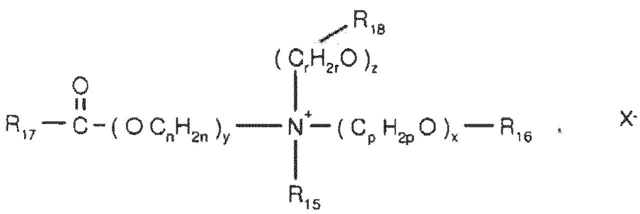 (VIII)

worin:

- $R_{15}$ aus $C_1$-$C_6$-Alkyl-, $C_1$-$C_6$-Hydroxyalkyl- und $C_2$-$C_6$-Dihydroxyalkylresten ausgewählt ist;
- $R_{16}$ aus dem Rest $R_{19}$-CO-, einem linearen oder verzweigten Alkyl- oder Alkenylrest $R_{20}$ mit 1 bis 22 Kohlenstoffatomen und einem Wasserstoffatom ausgewählt ist,
- $R_{18}$ aus dem Rest $R_{21}$-CO-, einem linearen oder verzweigten Alkyl- oder Alkenylrest $R_{22}$ mit 1 bis 6 Kohlenstoffatomen und einem Wasserstoffatom ausgewählt ist,
- $R_{17}$, $R_{19}$ und $R_{21}$ gleich oder verschieden sind und aus linearen oder verzweigten Alkyl- oder Alkenylresten mit 12 bis 22 Kohlenstoffatomen ausgewählt sind;
- n, p und r gleich oder verschieden sind und für ganze Zahlen im Bereich von 2 bis 6 stehen;
- y für eine ganze Zahl im Bereich von 1 bis 10 steht;
- x und z gleich oder verschieden sind und für ganze Zahlen im Bereich von 0 bis 10 stehen;
- $X^-$ ein einfaches oder komplexes organisches oder anorganisches Anion ist;

mit der Maßgabe, dass die Summe x + y + z 1 bis 15 beträgt, dann, wenn x gleich 0 ist, $R_{16}$ für $R_{20}$ steht und dann, wenn z gleich 0 ist, $R_{18}$ für $R_{22}$ steht;
(v) Mischungen davon

ausgewählt ist.

**7.** Zusammensetzung nach einem der vorhergehenden Ansprüche, **dadurch gekennzeichnet, dass** das kationische Tensid die Formel (V) und vorzugsweise die Formel (V) Option (1) aufweist.

**8.** Zusammensetzung nach einem der vorhergehenden Ansprüche, **dadurch gekennzeichnet, dass** der Gehalt an kationischem Tensid bzw. kationischen Tensiden zwischen 0,01 und 25 Gew.-%, bezogen auf das Gewicht der Zusammensetzung, liegt.

**9.** Zusammensetzung nach einem der vorhergehenden Ansprüche, **dadurch gekennzeichnet, dass** das Gewichtsverhältnis von Fettalkohol(en) zu Fettester(n) zwischen 3 und 9 liegt.

**10.** Zusammensetzung nach einem der vorhergehenden Ansprüche, **dadurch gekennzeichnet, dass** sie außerdem ein oder mehrere Oxidationsmittel umfasst.

**11.** Zusammensetzung nach einem der vorhergehenden Ansprüche, **dadurch gekennzeichnet, dass** sie einen pH-Wert zwischen 8 und 12 ausschließlich, vorzugsweise zwischen 9 und 11 ausschließlich aufweist.

**12.** Verfahren zum Färben von menschlichen Keratinfasern, **dadurch gekennzeichnet, dass** man auf die trockenen oder feuchten Keratinfasern eine durch unmittelbares Mischen einer Färbezusammensetzung (A) nach einem der Ansprüche 1 bis 9 mit mindestens einer Zusammensetzung, die ein oder mehrere Oxidationsmittel umfasst, vor dem Aufbringen erhaltene gebrauchsfertige Zusammensetzung (B) aufbringt.

**13.** Verfahren zum Färben von menschlichen Keratinfasern, **dadurch gekennzeichnet, dass** man die Zusammensetzung (A) nach einem der Ansprüche 1 bis 9 in Gegenwart einer oxidierenden Zusammensetzung aufbringt, wobei die oxidierende Zusammensetzung vor oder nach der Zusammensetzung (A) ohne Zwischenspülung aufgebracht wird.

**14.** Vorrichtung mit mehreren Kompartimenten, umfassend in mindestens einem ersten Kompartiment die Zusammensetzung (A) nach einem der Ansprüche 1 bis 9 und in mindestens einem zweiten Kompartiment eine Zusammensetzung, die ein oder mehrere Oxidationsmittel umfasst.

## Claims

**1.** Dyeing composition (A) for colouring human keratin fibres, containing, at a pH greater than or equal to 8, in a cosmetically acceptable medium,

* one or more oxidative dye precursors;
* one or more cationic surfactants;
* one or more fatty acid esters selected from the esters of saturated linear $C_1$-$C_{18}$ monohydric alcohols, with linear, branched, saturated or unsaturated, $C_{14}$-$C_{18}$ monofunctional fatty acids;
* one or more fatty alcohols,
* the weight ratio of fatty alcohol(s) to fatty acid ester (s) being greater than 2 and less than 10;
* the weight ratio of fatty alcohol(s) to cationic surfactant(s) being between 2 and 30.

**2.** Composition according to the preceding claim, **characterized in that** the composition includes one or more direct dyes.

**3.** Composition according to any one of the preceding claims, **characterized in that** the content of fatty ester(s) is between 0.3 and 12.5 wt.%, relative to the weight of the composition.

**4.** Composition according to any one of the preceding claims, **characterized in that** the fatty alcohol is selected from the non-(poly)oxyalkylenated and non-(poly)glycerolated monohydric alcohols, having from 14 to 22 carbon atoms, preferably having from 16 to 18 carbon atoms, saturated.

**5.** Composition according to any one of the preceding claims, **characterized in that** the content of fatty alcohol(s) is between 3 and 25 wt.%, relative to the weight of the composition.

**6.** Composition according to any one of the preceding claims, **characterized in that** the cationic surfactant is selected from:

(i) the quaternary ammonium salts of the following formula (V):

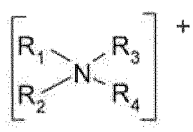 $X^-$ (V)

in which

$X^-$ is an anion and

(1) the radicals $R_1$ to $R_3$, which may be identical or different, represent an aliphatic, linear or branched, $C_1$-$C_4$ radical, or an aryl or alkaryl radical, the alkyl radical optionally bearing an alkoxy, alkyl amide

group,
$R_4$ denotes a linear or branched, $C_{16}$-$C_{30}$ alkyl radical; or
(2) the radicals $R_1$ and $R_2$, which may be identical or different, represent an aliphatic, linear or branched, $C_1$-$C_4$ radical, or an aryl or alkaryl radical, the alkyl radical optionally bearing an alkoxy, alkyl amide or hydroxyalkyl, $C_1$-$C_4$ group; $R_3$ and $R_4$, which may be identical or different, denote a linear or branched, $C_{12}$-$C_{30}$ alkyl or alkenyl radical, said radical optionally having at least one ester or amide function;

(ii) the quaternary ammonium imidazolinium salts of the following formula (VI):

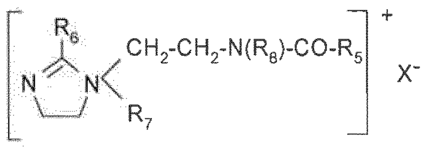
(VI)

in which

$R_5$ represents a $C_{12}$-$C_{30}$ alkenyl or alkyl radical,
$R_6$ represents a hydrogen atom, a $C_1$-$C_4$ alkyl radical or a $C_{12}$-$C_{30}$ alkenyl or alkyl radical,
$R_7$ represents a $C_1$-$C_4$ alkyl radical, $R_8$ represents a hydrogen atom, a $C_1$-$C_4$ alkyl radical,
X is an anion;

(iii) the quaternary diammonium salts of formula (VII) :

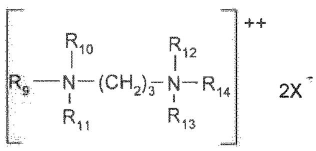
(VII)

in which

$R_9$ denotes a $C_{12}$-$C_{30}$ alkyl or alkenyl radical,
$R_{10}$, $R_{11}$, $R_{12}$, $R_{13}$ and $R_{14}$, which may be identical or different, are selected from hydrogen or a $C_1$-$C_4$ alkyl radical, and X is an anion;

(iv) the quaternary ammonium salts containing at least one ester function of the following formula (VIII):

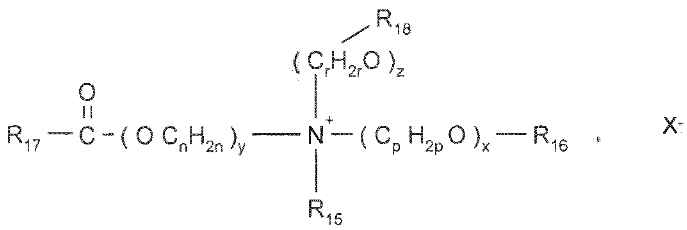
(VIII)

in which:

- $R_{15}$ is selected from the $C_1$-$C_6$ alkyl, $C_1$-$C_6$ hydroxyalkyl, $C_2$-$C_6$ dihydroxyalkyl radicals;
- $R_{16}$ is selected from the radical $R_{19}$-CO-, a radical $R_{20}$ alkyl or alkenyl $C_1$-$C_{22}$ linear or branched, a hydrogen atom,
- $R_{18}$ is selected from the radical $R_{21}$-CO-, a radical $R_{22}$ alkyl or alkenyl $C_1$-$C_6$ linear or branched, a hydrogen atom,
- $R_{17}$, $R_{19}$ and $R_{21}$, which may be identical or different, are selected from the linear or branched $C_{12}$-$C_{22}$ alkyl or alkenyl radicals;
- n, p and r, which may be identical or different, are integers with a value from 2 to 6;
- y is an integer with a value from 1 to 10;

- x and z, which may be identical or different, are integers with a value from 0 to 10;
- $X^-$ is an organic or inorganic simple or complex anion;

provided that the sum x + y + z has a value from 1 to 15, and that when x has a value 0 then $R_{16}$ denotes $R_{20}$ and that when z has a value 0 then $R_{18}$ denotes $R_{22}$;
(v) or mixtures thereof.

**7.** Composition according to any one of the preceding claims, **characterized in that** the cationic surfactant is of formula (V), preferably of formula (V) option (1).

**8.** Composition according to any one of the preceding claims, **characterized in that** the content of cationic surfactant(s) is between 0.01 and 25 wt.%, relative to the weight of the composition.

**9.** Composition according to any one of the preceding claims, **characterized in that** the weight ratio of fatty alcohol(s) to fatty ester(s) is between 3 and 9.

**10.** Composition according to any one of the preceding claims, **characterized in that** it contains one or more oxidizing agents.

**11.** Composition according to any one of the preceding claims, **characterized in that** it has a pH between 8 and 12 exclusive, preferably between 9 and 11 exclusive.

**12.** Method of colouring human keratin fibres, **characterized in that** a ready-to-use composition (B) obtained by extemporaneous mixing, before application, of a dyeing composition (A) according to any one of Claims 1 to 9, with at least one composition containing one or more oxidizing agents, is applied on dry or damp keratin fibres.

**13.** Method of colouring human keratin fibres, **characterized in that** composition (A) according to any one of Claims 1 to 9 is applied in the presence of an oxidizing composition; the oxidizing composition being applied before or after said composition (A), without intermediate rinsing.

**14.** Device with several compartments containing, in at least one first compartment, composition (A) according to any one of Claims 1 to 9, and in at least one second compartment, a composition comprising one or more oxidizing agents.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136
- WO 9515144 A
- WO 9501772 A
- EP 714954 A
- FR 2189006
- FR 2285851
- FR 2140205
- EP 1378544 A
- EP 1674073 A
- EP 1637566 A
- EP 1619221 A
- EP 1634926 A
- EP 1619220 A
- EP 1672033 A
- EP 1671954 A
- EP 1671955 A
- EP 1679312 A
- EP 1671951 A
- EP 167952 A
- EP E167971 A
- WO 06063866 A
- WO 06063867 A
- WO 06063868 A
- WO 06063869 A
- EP 1408919 A
- EP 1377264 A
- EP 1377262 A
- EP 1377261 A
- EP 1377263 A
- EP 1399425 A
- EP 1399117 A
- EP 1416909 A
- EP 1399116 A
- EP 1671560 A
- EP 1006153 A
- EP 1433472 A
- EP 1433474 A
- EP 1433471 A
- EP 1433473 A
- EP 6291333 A
- EP 557203 A
- FR 2270846
- US 4157388 A
- US 4390689 A
- US 4702906 A
- US 4719282 A
- EP 0557203 A


### Littérature non-brevet citée dans la description


- COLOUR INDEX INTERNATIONAL
- **RICHARD J. CRAWFORD.** *Journal of the Society of Cosmetic Chemists,* 1980, vol. 31 (5), 273-278
- CTFA. 1993